(19) Europäisches Patentamt
European Patent Office
Office européen des brevets

(11) Veröffentlichungsnummer : **0 062 919**
**B1**

(12) # EUROPÄISCHE PATENTSCHRIFT

(45) Veröffentlichungstag der Patentschrift : **30.07.86**

(51) Int. Cl.⁴ : **C 07 D209/08**, C 07 D209/42, A 61 K 31/40

(21) Anmeldenummer : **82103106.9**

(22) Anmeldetag : **13.04.82**

(54) **Neue Indol-Derivate, Verfahren zu ihrer Herstellung und pharmazeutische Präparate, die diese Verbindungen enthalten.**

(30) Priorität : **13.04.81 DE 3115993**

(43) Veröffentlichungstag der Anmeldung : **20.10.82 Patentblatt 82/42**

(45) Bekanntmachung des Hinweises auf die Patenterteilung : **30.07.86 Patentblatt 86/31**

(84) Benannte Vertragsstaaten : **AT BE CH DE FR GB IT LI LU NL SE**

(56) Entgegenhaltungen :
**WO-A-80 /001 52**
**FR-A- 1 532 210**
**FR-A- 2 370 472**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Heindl, Josef, Dr.**
**Eitel-Fritz-Strasse 40**
**D-1000 Berlin 38 (DE)**
Erfinder : **Loge, Olaf, Dr.**
**Bekassinenweg 37**
**D-1000 Berlin 27 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neue Indol-Derivate der allgemeinen Formel I gemäß Patentanspruch 1, ein Verfahren zu ihrer Herstellung gemäß Patentanspruch 41 und pharmazeutische Präparate gemäß Patentanspruch 40, die diese Verbindungen enthalten.

Als Alkylgruppen $R_2$, $R_4$ und $R_5$ der neuen Indol-Derivate seien beispielsweise genannt: Die Methylgruppe, die Ethylgruppe, die sec.-Butylgruppe, die Isobutylgruppe, die tert.-Butylgruppe, die n-Pentylgruppe, die Isoamylgruppe und die n-Hexylgruppe.

Ester der Hydroxymethylgruppe $R_2$ sind beispielsweise die Acetate, Propionate, Butyrate, Isobutyrate, Valerianate oder Capronate dieser Gruppen.

Als Cycloalkylreste $R_2$ und $R_5$ seien beispielsweise genannt, der Cyclopentylrest, der Cyclopentylmethylrest, der Cyclohexylrest, der Cyclopropylrest oder der Cyclobutylrest.

Die Erfindung betrifft sowohl die racemischen Indol-Derivate der allgemeinen Formel I gemäß Patentanspruch 1 als auch deren optische Antipoden.

Indol-Derivate, die als Wirkstoffe in anerkannt wirksamen Handelspräparaten enthalten sind, sind vorbekannt, so zum Beispiel das Pindolol, dies ist das 1-(4-Indolyloxy)-3-(isopropylamino)-2-propanol oder das Mepindolol, dies ist das 1-[4-(2-methylindolyl)-oxy]-3-isopropylamino-2-propanol. Diese Wirkstoffe, die eine gewisse Stukturanalogie zu den erfindungsgemäßen Indol-Derivaten zeigen sind bekanntlich β-Receptorenblocker.

Demgegenüber sind die erfindungsgemäßen Indol-Derivate der allgemeinen formel I — insbesondere die Ester dieser allgemeinen Formel — überraschenderweise β-Receptoren-Stimmulatoren und haben darüberhinaus auch eine antiallergische, antiphlogistische und analgetische Wirksamkeit. Die freien Säuren der allgemeinen Formel I und deren Alkalimetallsalze oder Erdalkalimetallsalze sind meist weniger wirksam ; sie eignen sich unter anderem auch als Zwischenprodukte zur Synthese hochwirksamer Ester.

Aufgrund ihrer β-rezeptorenstimmulierenden Aktivität verursachen die erfindungsgemäßen Indol-Derivate eine Dilatation der Bronchien und sind demzufolge zur Behandlung von Erkrankungen der Atemwege, wie zum Beispiel des Bronchialasthmas geeignet und bewirken eine Tokolyse, so daß sie zur Verhinderung vorzeitig einsetzender Wehen eingesetzt werden können.

Die Verbindungen können oral, durch Injektion, Infusion oder Inhalation verabreich werden.

Zur Herstellung der Inhalationsmittel können die Indol-Derivate in üblicher Weise pulverisiert oder in einem geeigneten Lösungsmittel gelöst oder suspendiert und mit geeigneten Zusätzen, wie Verdünnungsmitteln, Suspensionshilfsmitteln, Treibgasen, Geschmackskorregentien etc. versetzt werden.

Es ist selbstverständlich, daß den so hergestellten Inhalationsmitteln zusätzlich noch andere Wirkstoffe, wie zum Beispiel Antibiotika zugesetzt werden können.

Für die orale Anwendung eignen sich beispielsweise Tabletten, Dragees und Kapseln, welche neben dem Wirkstoff einen pharmakologisch unwirksamen Träger, wie zum Beispiel Laktose, Amylose, Talkum, Gelatine, Magnesiumstearat und ähnliches, sowie die üblichen Zusätze enthalten.

Die erfindungsgemäßen Indol-Derivate können nach den im Patentanspruch 41 gekennzeichneten Verfahren hergestellt werden. Diese Reaktion kann beispielsweise in der Weise durchgeführt werden, daß man die Verbindungen der allgemeinen Formel II in einem inerten Lösungsmittel in Gegenwart von Palladium- oder Platinkatalysatoren hydriert.

Andererseits ist es aber auch möglich, die Verbindungen zum Beispiel mit komplexen Metallhydriden, die nicht befähigt sind Carboxylgruppen oder Alkoxycarbonylgruppen wie zum Beispiel Natriumborhybrid, zu reduzieren. Will man bevorzugt einen optischen Antipoden des Racemates darstellen, kann es zweckmäßig sein, eine der asymmetrischen Reduktionen durchzuführen, die in Houben-Weyl, Methoden der organischen Chemie, 4. Auflage, 1955 Band IV/2 Seite 535 ff beschrieben sind.

Die meist unbekannten Ausgangsverbindungen der allgemeinen Formel II können auf unterschiedlichem Wege hergestellt werden, so zum Beispiel aus Indol-Derivaten der allgemeinen Formel III

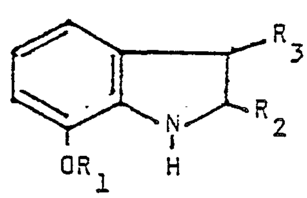

(III)

worin $R_1$, $R_2$ und $R_3$ die obengenannte Bedeutung besitzen.

So kann man beispielsweise diese Verbindungen unter den üblichen Bedingungen der Hoesch-Synthese mit einem Nitril der allgemeinen Formel IV

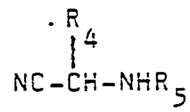

(IV)

worin $R_4$ und $R_5$ die obengenannte Bedeutung besitzen, kondensieren, indem man beispielsweise die Verbindungen der Formel III und das Nitril in Gegenwart von Lewis Säuren in einem inerten Lösungsmittel unter Einleiten von Chlorwasserstoff umsetzt.

Andererseits kann man die Indol-Derivate der allgemeinen Formel III unter den Bedingungen der Friedel-Crafts-Acylierung (Beispielsweise in einem inerten Lösungsmittel in Gegenwart von Aluminiumchlorid) mit einem α-Halogen-alkanoylhalogenid der allgemeinen Formel V

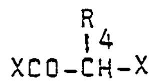

(V)

worin X vorzugsweise ein Chloratom, oder ein Bromatom bedeutet, umsetzen und die so dargestellte Verbindung der allgemeinen Formel VI

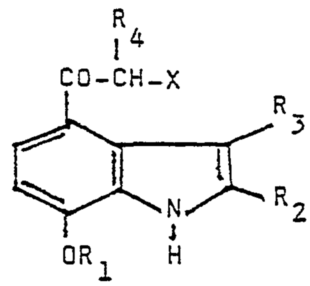

(VI)

worin $R_1$, $R_2$, $R_3$, $R_4$ und X die obengenannte Bedeutung besitzen, miteinem Amin der allgemeinen Formel VII

$$H_2N—R_5 \quad \text{(VII)},$$

kondensiert.

Die nachfolgenden Ausführungsbeispiele dienen zur Erläuterung des erfindungsgemäßen Verfahrens und zur Erläuterung der Herstellungsverfahren der Verbindungen der allgemeinen Formel II, die Ausgangssubstanzen für das erfindungsgemäße Verfahren sind.

Beispiel 1

A. Zu einer Lösung von 56,7 g 7-Methoxyindol-2-carbonsäure in 300 ml Methanol gibt man 250 ml Chlorwasserstoff-gesättigtes Methanol und erhitzt 2 Stunden lang unter Rückfluß. Dann kühlt man die Lösung im Eis-Methanol-Bad stark ab, saugt die abgeschiedenen Kristalle ab und wäscht sie mit wenig kaltem Methanol. Man erhält so 26,5 g 7-Methoxyindol-2-carbonsäure-methylester vom Schmelzpunkt 118-119 °C.

B. Eine Lösung von 42,7 g 7-Methoxyindol-2-carbonsäure-methylester in 950 ml Dichlormethan wird unter Stickstoff und Rühren auf —65 °C gekühlt. Dann tropft man bei dieser Temperatur 65 ml Bortribromid in die Lösung, rührt noch 15 Minuten bei dieser Temperatur, läßt die Reaktionsmischung sich auf Raumtemperatur erwärmen und rührt noch eine weitere Stunde. Dann rührt man die Mischung in 800 ml Eiswasser, trennt die organische Phase ab, extrahiert die wässrige Phase nochmal mit Äthylacetat und trocknet die vereinigten organischen Phasen mit Natriumsulfat. Dann engt man sie im Vakuum zur Trockne ein, kristallisiert den Rückstand aus Acetonitril um und erhält 31,0 g 7-Hydroxyindol-2-carbonsäure-methylester vom Schmelzpunkt 218-220 °C.

C. Zu einer auf 10 °C gekühlten Lösung von 5,6 g Aluminium-Chlorid in 20 ml Nitrobenzol gibt man 1,9 g 7-Hydroxyindol-2-carbonsäure-methylester und 925 mg Aminoacetonitril-hydrochlorid und kühlt dann die Mischung auf 5 bis 10 °C. Bei dieser Temperatur leitet man 8 Stunden lang Chlorwasserstoff-Gas in die Reaktionsmischung, läßt sie über Nacht stehen und gießt sie dann in Eiswasser. Nach 10 Minuten saugt man den abgeschiedenen Niederschlag ab, kristallisiert das Rohprodukt aus 2 n Salzsäure/Methanol 1/2 um und erhält 1,8 g 4-Aminoacetyl-7-hydroxyindol-2-carbonsäure-methylester-Hydrochlorid vom Schmelzpunkt oberhalb 280 °C.

D. Zu einer Lösung von 1,14 g 4-Aminoacetyl-7-hydroxyindol-2-carbonsäure-methylester-Hydrochlorid in 200 ml Methanol gibt man 110 mg 10 %igen Palladium-Katalysator auf Aktivkohle und hydriert 3 Stunden lang unter Schütteln bei Normaldruck. Dann filtriert man die Reaktionsmischung und engt das Filtrat zur Trockne ein. Der Rückstand wird mit Aceton/Methanol 9/1 kräftig gerührt, das erhaltene Produkt abfiltriert und im Vakuum getrocknet. Man erhält so 1,0 g 4-(2-Amino-1-hydroxyäthyl)-7-hydroxyindol-2-carbonsäure-methylester-Hydrochlorid vom Zersetzungspunkt 280 °C.

Beispiel 2

A. Zu einer Lösung von 8,4 g Aluminiumchlorid in 30 ml Nitrobenzol gibt man bei 0 °C 1,7 g Methylaminoacetonitril-Hydrochlorid und 3,0 g 7-Hydroxyindol-2-carbonsäure-methylester und leitet dann in die erhaltene Lösung bei —4 bis —2 °C 6 Stunden lang Chlorwasserstoffgas ein. Dann rührt man die Reaktionsmischung 16 Stunden lang bei der gleichen Temperatur, gießt sie in Eiswasser, rührt noch 10 Minuten lang und saugt den Niederschlag ab, welcher verworfen wird. Das Filtrat wird mit Petroläther versetzt, der Niederschlag abgesaugt, mit Äthanol ausgekocht und vom Ungelösten abgesaugt. Man erhält 1,1 g 4-Methylaminoacetyl-7-hydroxyindol-2-carbonsäure-methylester-Hydrochlorid vom Zersetzungspunkt 255-256 °C.

B. Eine Lösung von 896 mg 4-Methylaminoacetyl-7-hydroxyindol-2-carbonsäure-methylester-Hydrochlorid in 30 ml Methanol wird mit 150 mg 10 %igem Palladium-Tierkohle-Katalysator versetzt und unter Schütteln bei Normaldruck hydriert. Dann filtriert man die Reaktionsmischung und engt das Filtrat im Vakuum zur Trockne ein. Der Rückstand wird mit 20 ml Acetonitril ausgekocht und der unlösliche Anteil abgesaugt und aus Äthanol umkristallisiert. Man erhält so 232 mg 4-(1-Hydroxy-2-methylaminoäthyl)-7-hydroxyindol-2-carbonsäure-methylester-Hydrochlorid vom Zersetzungspunkt 177-180 °C.

Beispiel 3

A. Eine Lösung von 11,2 g Aluminiumchlorid in 40 ml Nitrobenzol wird mit 2,8 g Isopropylaminoacetonitril-Hydrochlorid und 4,0 g 7-Hydroxyindol-2-carbonsäure-methylester versetzt. Dann leitet man in die Mischung bei 0 °C 4 1/2 Stunden lang Chlorwasserstoff-Gas ein, rührt die Mischung noch 16 Stunden lang bei 0 °C und gießt sie in Eiswasser. Man saugt den Niederschlag ab, wäscht ihn mit Wasser und Petroläther und trocknet ihn. Das erhaltene Produkt wird in wenig Aceton suspendiert, abgesaugt, mit Diäthylät her gewaschen und mit 50 ml Äthanol aufgekocht. Das Ungelöste wird abgesaugt, mit kaltem Äthanol gewaschen, getrocknet und man erhält 1,18 g 7-Hydroxy-4-isopropylaminoacetylindol-2-carbonsäure-methylester-Hydrochlorid vom Zersetzungspunkt 273 °C.

B. Eine Lösung von 1,6 g 7-Hydroxy-4-isopropylaminoacetylindol-2-carbonsäure-methylester-Hydrochlorid in 80 ml Methanol wird mit 300 mg 10 %iger Palladium-Tierkohle versetzt und 2 1/4 Stunden lang unter Normaldruck mit Wasserstoff geschüttelt. Dann filtriert man die Reaktionsmischung und engt das Filtrat im Vakuum zur Trockne ein. Der Rückstand wird mit Acetonitril verrieben, auf 0 °C gekühlt, filtriert und man erhält 1,41 g 4-(1-Hydroxy-2-isopropylamino-äthyl)-7-hydroxyindol-2-carbonsäure-methylester vom Zersetzungspunkt 137-139 °C.

Beispiel 4

658 mg 4-(1-Hydroxy-2-isopropylamino-äthyl)-7-hydroxyindol-2-carbonsäure-methylester-Hydrochlorid werden in 20 ml 1 n Natronlauge gelöst und 45 Minuten lang bei Raumtemperatur gerührt. Anschließend kühlt man die Reaktionsmischung im Eisbad, säuert sie mit konz. Essigsäure an, rührt noch 10 Minuten, saugt das Kristallisat ab und wäscht es mit Wasser und Äthanol. So erhält man 383 mg 4-(1-Hydroxy-2-isopropylamino)-7-hydroxyindol-2-carbonsäure vom Zersetzungspunkt oberhalb 173 °C.

Beispiel 5

Unter den Bedingungen des Beispiels 3 A werden 10,0 g 7-Hydroxyindol-2-carbonsäure-methylester und 7,8 g tert.-Butylaminoacetonitril-Hydrochlorid umgesetzt, aufbereitet und man erhält 4,8 g 4-tert.-Butylaminoacetyl-7-hydroxyindol-2-carbonsäure-methylester-Hydrochlorid vom Zersetzungspunkt 271 °C.

3,4 g des erhaltenen Produkts werden unter den Bedingungen des Beispiels 3 B hydriert, aufbereitet und man erhält 3,0 g 4-(2-tert.-Butylamino-1-hydroxy-äthyl)-7-hydroxyindol-2-carbonsäure-methylester-Hydrochlorid vom Zersetzungspunkt 168-173 °C.

Beispiel 6

Unter den Bedingungen des Beispiels 4 werden 500 mg 4-(tert.-Butylamino-1-hydroxyäthyl)-7-hydroxyindol-2-carbonsäure-methylester-Hydrochlorid hydrolysiert, aufbereitet, und man erhält 435 mg 4-(2-tert.-Butylamino-1-hydroxyäthyl)-7-hydroxyindol-2-carbonsäure vom Zersetzungspunkt 260-261 °C.

Beispiel 7

A. Zu einer Suspension von 31,3 g Kaliumäthylat in 700 ml Diäthyläther werden 181 g Oxalsäurediäthylester eingetropft. Dann setzt man der Reaktionsmischung portionsweise 51,7 g 3-Methyl-2-nitroanisol zu und erhitzt die Mischung 18 Stunden lang unter Rückfluß. Man läßt erkalten, saugt den

erhaltenen Niederschlag ab, wäscht ihn mit Diäthyläther und löst ihn in einer Mischung aus 650 ml Äthanol und 650 ml konz. Essigsäure. Dieser Lösung setzt man 167 g Eisenpulver zu, und erhitzt die Mischung 90 Minuten lang unter Rückfluß.

Man läßt die Reaktionsmischung erkalten und gießt sie in 5 l Eiswasser. Man filtriert die Mischung über Kieselgur, wäscht dieses mit Diäthyläther und Äthylacetat, trennt die organische Phase ab und extrahiert die wässrige Phase noch dreimal mit Äthylacetat. Die vereinigten organischen Phasen werden mit gesättigter Natriumcarbonatlösung gewaschen, getrocknet und im Vakuum eingeengt. Der Rückstand wird aus Äthanol umkristallisiert und man erhält 32,4 g 7-Methoxyindol-2-carbonsäure-äthylester vom Schmelzpunkt 113 °C.

B. 11,0 g 7-Methoxyindol-2-carbonsäure-äthylester werden unter den Bedingungen des Beispiels 1 B mit Bortribromid umgesetzt, aufbereitet und man erhält nach Umkristallisation aus Äthanol 4,7 g 7-Hydroxyindol-2-carbonsäure-äthylester vom Schmelzpunkt 195-200 °C.

C. Unter den Bedingungen des Beispiels 3 A werden 4,1 g 7-Hydroxyindol-2-carbonsäure-äthylester mit 3,0 g tert.-Butylaminoacetonitril-Hydrochlorid umgesetzt, aufbereitet und man erhält 1,75 g 4-tert.-Butylaminoacetyl-7-hydroxyindol-2-carbonsäure-äthylester-Hydrochlorid vom Zersetzungspunkt 272 °C.

D. Unter den Bedingungen des Beispiels 1 D werden 1,0 g 4-tert.-Butylaminoacetyl-7-hydroxyindol-2-carbonsäure-äthylester-Hydrochlorid umgesetzt, aufbereitet und man erhält nach Umkristallisation aus Äthanol 573 mg 4-(2-tert.-Butylamino-1-hydroxyäthyl)-7-hydroxyindol-2-carbonsäureäthylester-Hydrochlorid vom Zersetzungspunkt 168-170 °C.

Beispiel 8

A. Zu einer Suspension von 6,0 g 7-Methoxyindol-2-carbonsäure in 100 ml Toluol werden 4,3 ml Thionylchlorid zugefügt und man erhitzt die Mischung unter Rühren 3 Stunden lang auf 90 °C. Dann engt man das Reaktionsgemisch im Vakuum ein, versetzt den Rückstand mit 50 ml n-Butanol und erhitzt eine Stunde lang unter Rückfluß. Anschließend engt man die Mischung im Vakuum zur Trockne ein, kocht den öligen Rückstand mit 60 ml Petroläther auf, dekantiert die klare Lösung vom dunklen Rückstand ab und erhält nach Abkühlen derselben 4,1 g 7-Methoxyindol-2-carbonsäure-n-butylester vom Schmelzpunkt 66 bis 69 °C.

B. 4,1 g 7-Methoxyindol-2-carbonsäure-n-butylester werden unter den Bedingungen des Beispiels 1 B mit Bortribromid umgesetzt, aufbereitet und man erhält nach Umkristallisation aus Diisopropyläther 2,1 g 7-Hydroxyindol-2-carbonsäure-n-butylester vom Schmelzpunkt 142-144 °C.

C. Zu einer Lösung von 2,7 g Aluminiumchlorid in 20 ml Nitrobenzol werden unter Eiskühlung und Rühren 818 mg tert.-Butylaminoacetonitril-Hydrochlorid und 1,2 g 7-Hydroxyindol-2-carbonsäure-n-butylester gegeben. Dann leitet man in die Mischung bei 0 bis 3 °C 7 Stunden lang Chlorwasserstoff-Gas ein, rührt die Reaktionsmischung noch 16 Stunden lang bei 0 bis 3 °C und gießt sie dann in Eiswasser. Man rührt das Gemisch 10 Minuten lang und saugt dann den Niederschlag ab. Dieser wird aus Isopropanol umkristallisiert und man erhält 765 mg 4-tert.-Butylaminoacetyl-7-hydroxyindol-2-carbonsäure-n-butylester-Hydrochlorid vom Zersetzungspunkt 203-208 °C.

D. Unter den Bedingungen des Beispiels 3 B werden 766 mg 4-tert.-Butylaminoacetyl-7-hydroxyindol-2-carbonsäure-n-butylester-Hydrochlorid hydriert, aufbereitet und man erhält 640 mg 4-(2-tert.-Butylamino-1-hydroxyäthyl)-7-hydroxyindol-2-carbonsäure-n-butylester-Hydrochlorid vom Zersetzungspunkt 148-149 °C.

Beispiel 9

A. Unter den Bedingungen des Beispiels 8 A werden 8 g 7-Methoxyindol-2-carbonsäure in das Säurechlorid überführt und dieses mit Isobutanol umgesetzt und man erhält nach Aufarbeitung und Umkristallisation aus Petroläther 6,2 g 7-Methoxy-indol-2-carbonsäure-isobutylester vom Schmelzpunkt 100-101 °C.

B. Unter den Bedingungen des Beispiels 1 B werden 5 g 7-Methoxyindol-2-carbonsäure-isobutylester umgesetzt, aufbereitet und man erhält nach Umkristallisation aus Isobutanol 2,23 g 7-Hydroxyindol-2-carbonsäure-isobutylester vom Schmelzpunkt 185-187 °C.

C. 1,0 g 7-Hydroxyindol-2-carbonsäure-isobutylester werden unter den Bedingungen des Beispiels 8 C mit 682 mg tert.-Butylaminoacetonitril-Hydrochlorid umgesetzt, aufbereitet und man erhält 728 mg 4-tert.-Butylaminoacetyl-7-hydroxyindol-2-carbonsäure-isobutylester-Hydrochlorid vom Zersetzungspunkt 220-225 °C.

D. Unter den Bedingungen des Beispiels 3 B werden 1,1 g 4-tert.-Butylaminoacetyl-7-hydroxyindol-2-carbonsäure-isobutylester-Hydrochlorid hydriert, aufbereitet und man erhält 960 mg 4-(2-tert.-Butylamino-1-hydroxyäthyl)-7-hydroxyindol-2-carbonsäure-isobutylester-Hydrochlorid, das sich ab 135 °C zersetzt.

Beispiel 10

A-1. 10,3 g 7-Methoxyindol-2-carbonsäure-methylester werden unter den Bedingungen des Beispiels 1 C mit 7,5 g tert.-Butylaminoacetonitril-Hydrochlorid umgesetzt, aufbereitet, und man erhält nach Umkristallisation aus 1,2-Dimethoxy-äthan 8,0 g 4-tert.-Butylaminoacetyl-7-methoxyindol-2-carbonsäure-methylester-Hydrochlorid vom Zersetzungspunkt 189-191 °C.

A-2. Zu einer Lösung von 1,02 g 7-Methoxyindol-2-carbonsäure-methylester in 25 ml Dichlormethan werden 2 g Aluminiumchlorid und 0,65 ml Bromacetylbromid gegeben und die Mischung 3 Stunden lang unter Rückfluß erhitzt. Man läßt die Reaktionsmischung erkalten und gießt sie in 100 ml eines Gemischs aus gleichen Teilen Eiswasser und konz. Salzsäure. Man trennt die organische Phase ab engt sie im Vakuum zur Trockne ein und erhält nach Umkristallisation aus Acetonitril 650 mg 4-Bromacetyl-7-methoxyindol-2-carbonsäure-methylester vom Schmelzpunkt 197-201 °C.

Zu einer Lösung von 300 mg 4-Bromacetyl-7-methoxyindol-2-carbonsäure-methylester in 5 ml Tetrahydrofuran gibt man 0,3 ml tert.-Butylamin und rührt die Mischung 4 Stunden lang bei Raumtemperatur. Dann saugt man das ausgefallene tert.-Butylamin-hydrochlorid ab und engt das Filtrat zur Trockne ein. Der Rückstand wird in 20 ml Dimethylformamid gelöst, auf 10 g Kieselgel aufgezogen und an 75 g Kieselgel mittels Toluol-Eisessig-Wasser 10 : 10 : 1 chromatographiert. Die das Verfahrensprodukt enthaltende Fraktion wird eingeengt, unter Erwärmen in 5 ml Wasser aufgenommen und mit konzentriertem Ammoniak alkalisch gemacht. Der erhaltene Niederschlag wird abgesaugt, in 5 ml Äthanol gelöst, bis zur Trübung mit ätherischer Salzsäure versetzt und auf ca. 0 °C gekühlt.
Man saugt das erhaltene Kristallisat ab, trocknet es und erhält 52 mg 4-tert.-Butylaminoacetyl-7-methoxyindol-2-carbonsäure-methylester-Hydrochlorid vom Zersetzungspunkt 187-189 °C.

B. Unter den Bedingungen des Beispiels 1 D werden 8,0 g 4-tert.-Butylaminoacetyl-7-methoxyindol-2-carbonsäure-methylester-Hydrochlorid hydriert, aufbereitet und man erhält 7,5 g 4-(2-tert.-Butylamino-1-hydroxyäthyl)-7-methoxyindol-2-carbonsäure-methylester-Hydrochlorid vom Zersetzungspunkt 193-194 °C.

Beispiel 11

A. Zu einer Lösung von 53 g Natriumhydrogensulfit in 125 ml Wasser werden 38 ml 37 %ige Formaldehydlösung gegeben und die Mischung eine Stunde lang bei 60-65 °C gerührt. Dann läßt man sie auf Raumtemperatur abkühlen, tropft 103,5 g 3-Benzylaminobuttersäure-methylester zu und rührt 2 Stunden lang bei Eigentemperatur (ca. 35 °C). Dann gibt man zur Reaktionsmischung eine Lösung von 25 g Natriumcyanid in 80 ml Wasser und rührt das Gemisch nochmals 2 Stunden lang und läßt es anschließend 16 Stunden lang bei Raumtemperatur stehen. Dann wird die Mischung mit Diathyläther extrahiert, der Ätherextrakt über Natriumsulfat getrocknet, das Lösungsmittel im Vakuum abgedampft und der Rückstand bei 0,3 torr destilliert. Man erhält 76 g 3-(N-Benzyl-N-cyanmethylamino)-buttersäure-methylester vom Siedepunkt 115-130 °C bei 0,3 torr.

B. Unter den Bedingungen des Beispiels 1 C werden 3,07 g 7-Hydroxyindol-2-carbonsäure-methylester mit 4,1 g 3-(N-Benzyl-N-cyanmethylamino)-buttersäure-methylester umgesetzt, aufbereitet und man erhält 1,35 g 4-[N-Benzyl-N-(2-methoxycarbonyl-1-methyläthyl)-aminoacetyl]-7-hydroxyindol-2-carbonsäure-äthylester-Hydrochlorid vom Zersetzungspunkt 215-220 °C.

C. Unter den Bedingungen des Beispiels 3 B werden 300 mg 4-[N-Benzyl-N-(2-methoxycarbonyl-1-methyläthyl)-aminoacetyl]-7-hydroxyindol-2-carbonsäure-äthylester-Hydrochlorid hydriert und man erhält 185 mg 4-[1-Hydroxy-2-(2-methoxycarbonyl-1-methyl-äthylamino)-äthyl]-7-hydroxyindol-2-carbonsäure-äthylester-Hydrochlorid, das sich oberhalb 100 °C zersetzt.

Beispiel 12

A. Zu einer Lösung von 106 g Natriumhydrogensulfit in 250 ml Wasser werden 44 g Acetaldehyd gegeben und die Mischung eine Stunde lang bei 60-65 °C gerührt. Dann läßt man sie auf Raumtemperatur abkühlen, tropft 73,1 g tert.-Butylamin zu und rührt 2 Stunden bei Raumtemperatur. Dann gibt man zur Reaktionsmischung eine Lösung von 150 g Natriumcyanid in 160 ml Wasser und rührt das Gemisch nochmals 2 Stunden lang und läßt es anschließend 16 Stunden lang bei Raumtemperatur stehen. Dann wird die Mischung mit Diäthyläther extrahiert, der Ätherextrakt über Natriumsulfat getrocknet, das

Lösungsmittel im Vakuum abgedampft und der Rückstand bei 14 torr destilliert. Man erhält 73,5 g 2-tert.-Butylaminopropionitril vom Siedepunkt 61-63 °C bei 14 torr.

B. Unter den Bedingungen des Beispiels 3 A werden 2,05 g 7-Hydroxyindol-2-carbonsäure-äthylester mit 1,38 g 2-tert.-Butylaminopropionitril umgesetzt, aufbereitet und man erhält 520 mg 4-(2-tert.-Butylamino)-propionyl-7-hydroxyindol-2-carbonsäure-äthylester-Hydrochlorid vom Zersetzungspunkt 180-185 °C.

C. Unter den Bedingungen des Beispiels 3 B werden 350 mg 4-(2-tert.-Butylamino)-propionyl-7-hydroxyindol-2-carbonsäure-äthylester-Hydrochlorid hydriert, aufbereitet und man erhält 300 mg 4-(2-tert.-Butylamino-1-hydroxypropyl)-7-hydroxyindol-2-carbonsäure-äthylester-Hydrochlorid vom Zersetzungspunkt 110-115 °C.

Beispiel 13

Unter den Bedingungen des Beispiels 3 A werden 2,05 g 7-Hydroxyindol-2-carbonsäure-äthylester mit 1,5 g Cyclohexylaminoacetonitril umgesetzt, aufbereitet und man erhält 1,0 g 4-Cyclohexylaminoacetyl-7-hydroxyindol-2-carbonsäure-äthylester-Hydrochlorid vom Zersetzungspunkt 256-259 °C.

B. Unter den Bedingungen des Beispiels 3 B werden 0,75 g 4-Cyclohexylaminoacetyl-7-hydroxyindol-2-carbonsäure-äthylester-Hydrochlorid umgesetzt, aufbereitet und man erhält 0,68 g 4-(2-Cyclohexylamino-1-hydroxyäthyl)-7-hydroxyindol-2-carbonsäure-äthylester-Hydrochlorid vom Zersetzungspunkt 180-182 °C.

Beispiel 14

a) Zu einer Lösung von 15,3 g 3-Methyl-2-nitrophenol in 150 ml Dimethylformamid werden 28 g Kaliumkarbonat und 34,2 g Benzylbromid gegeben und die Mischung 3 Stunden lang bei 70-80 °C gerührt. Danach werden die anorganischen Salze abgesaugt mit Dichlormethan gewaschen und das Filtrat im Vakuum eingeengt. Der Rückstand wird im Kugelrohr bei 120° Badtemperatur und 0,01 Torr destilliert und man erhält 23,2 g 3-Benzyloxy-2-nitrotoluol als Öl.

b) Zu einer Suspension von 9 g Kaliumethanolat in 200 ml Diethylether werden unter Rühren 26,3 g Oxalsäurediethylester und 15 Minuten später einer Lösung von 21,9 g 3-Benzyloxy-2-nitrotoluol in 30 ml Diethylether eingetropft. Man erhitzt die Reaktionsmischung 20 Stunden lang unter Rückfluß saugt das abgeschiedene Kaliumsalz des 3-Benzyloxy-2-nitrophenylbrenztraubensäureethylesters ab und wäscht es mit Diethylether. Dann löst man das Salz in einer Mischung aus 200 ml Ethanol und 200 ml konzentrierter Essigsäure, versetzt die Lösung mit 50 g Eisenpulver und erhitzt sie 90 Minuten lang unter Rückfluß. Man läßt das Reaktionsgemisch erkalten gießt es in 1 Liter Eiswasser, filtriert über Kieselgur und wascht das Kieselgur mit Diethylether. Die organische Phase wird abgetrennt und die wässrige Phase noch dreimal mit Diethylether extrahiert. Die vereinigten organischen Phasen werden mit gesättigter Natriumkarbonatlösung gewaschen, getrocknet und im Vakuum eingeengt. Der Rückstand wird aus Ethanol umkristallisiert und man erhält 9,6 g 7-Benzyloxyindol-2-carbonsäureethylester vom Schmelzpunkt 82-83 °C.

c) Zu einer Lösung von 22,2 g 7-Benzyloxyindol-2-carbonsäure-ethylester in 500 ml Ethanol gibt man 1 g 10 %igen Palladium-Katalysator auf Aktivkohle und hydriert 1 Stunde lang unter Schütteln bei Normaldruck. Dann filtriert man die Reaktionslösung und engt das Filtrat im Vakuum zur Trockne ein. Der Rückstand wird aus Ethanol umkristallisiert und man erhält 13,3 g 7-Hydroxyindol-2-carbonsäureethylester vom Schmelzpunkt 197-199 °C.

d) In eine Lösung von 840 mg Cyclopropylaminoacetonitril in 40 ml Dioxan wird unter Eiskühlung 15 Minuten lang Chlorwasserstoffgas eingeleitet. Dann fügt man 5,6 g Aluminiumchlorid zu, tropft in die Mischung eine Lösung von 2,05 g 7-Hydroxyindol-2-carbonsäureethylester in 20 ml Dioxan ein und leitet erneut unter Eiskühlung 2 Stunden lang Chlorwasserstoffgas ein. Dann rührt man die Reaktionsmischung 16 Stunden lang bei + 5 °C, gießt sie in Eiswasser rührt noch 20 Minuten und saugt den ausgefallenen Niederschlag ab. Der Niederschlag wird mit Ethanol ausgekocht, der verbleibende Rückstand getrocknet und man erhält 1,2 g 4-Cyclopropylaminoacetyl-7-hydroxyindol-2-carbonsäureethylester-Hydrochlorid vom Zersetzungspunkt 255-258 °C.

e) Zu einer Lösung von 504 mg 4-Cyclopropylaminoacetyl-7-hydroxyindol-2-carbonsaüreethylester-Hydrochlorid in 200 ml Methanol gibt man 100 mg 10 %igen Palladium-Katalysator auf Aktivkohle und hydriert zwei Stunden lang unter Schütteln bei Normaldruck. Dann filtriert man die Reaktionsmischung und engt das Filtrat zur Trockne ein. Der Rückstand wird mit 10 ml kaltem Wasser verrührt, das Ungelöste

abgesaugt und bei 50 °C im Vakuum getrocknet. Man erhält so 275 mg 4-(1-Cyclopropylamino-2-hydroxyethyl)-7-hydroxyindol-2-carbonsäureethylester-Hydrochlorid vom Zersetzungspunkt 195-197 °C.

Beispiel 15

Unter den Bedingungen des Beispiel 14 d werden 1,91 g 7-Hydroxyindol-2-carbonsäuremethylester und 2,4 g 2-(4-Methoxyphenyl)-1-methyl-ethylaminoacetonitril umgesetzt, aufbereitet und man erhält 1,55 g 4-[2-(4-Methoxyphenyl)-1-methyl-ethylaminoacetyl]-7-hydroxyindol-2-carbonsäuremethylester-Hydrochlorid vom Zersetzungspunkt 253-255 °C.

1 g dieses Produkts wird unter den Bedingungen des Beispiels 14 e hydriert, aufbereitet und man erhält 834 mg 4-{1-Hydroxy-2-[2-(4-methoxyphenyl)-1-methylethylamino]-ethyl}-7-hydroxyindol-2-carbonsäuremethylester-Hydrochlorid vom Zersetzungspunkt 120 °C.

Beispiel 16

300 mg 4-{1-Hydroxy-2-[2-(4-methoxyphenyl)-1-methylethylamino]-ethyl}-7-hydroxyindol-2-carbonsäuremethylester-Hydrochlorid werden in 5 ml n Natronlauge gelöst und 1 Stunde bei Raumtemperatur gerührt. Anschließend kühlt man die Reaktionsmischung im Eisbad, säuert sie mit konzentrierter Essigsäure an, rührt noch 10 Minuten, saugt das Kristallisat ab, wäscht es mit Wasser und Aceton, trocknet es im Vakuum und erhält 185 mg 4-{1-Hydroxy-2-[2-(4-methoxyphenyl)-1-methylethylamino]-ethyl}-7-hydroxyindol-2-carbonsäure vom Zersetzungspunkt 250 °C.

Beispiel 17

a) 20 g 3-Benzyloxy-2-nitrophenylbrenztraubensäureethylester werden in 100 ml Ethanol suspendiert, mit 28 g Methyljodid versetzt und zwei Stunden lang unter Rückfluß erhitzt. Man läßt die Reaktionsmischung erkalten, engt sie zur Trockne ein, verdünnt mit Wasser und extrahiert dreimal mit Diethylether.

Die organischen Phasen werden vereinigt, getrocknet und eingeengt. Der ölige Rückstand wird in einer Mischung aus 160 ml konzentrierter Essigsäure und 160 ml Ethanol gelöst, auf 60° erwärmt, portionsweise mit 36 g Eisenpulver versetzt und zwei Stunden lang bei 60-70 °C gerührt.

Man läßt die Reaktionsmischung über Nacht erkalten, dekantiert vom Eisenrückstand ab, extrahiert diesen noch dreimal mit Diethylether, vereinigt die organischen Phasen und trocknet sie. Nach Filtration über Aktivkohle engt man das Filtrat zur Trockne ein, kristallisiert den Rückstand aus Acetonitril um und erhält so 3,5 g 7-Benzyloxy-3-methylindol-2-carbonsäureethylester vom Schmelzpunkt 109-110 °C.

b) Unter den Bedingungen des Beispiels 14 c werden 1,3 g 7-Benzyloxy-3-methylindol-2-carbonsäureethylester hydriert, aufbereitet und man erhält 2,0 g 7-Hydroxy-3-methylindol-2-carbonsäurethylester vom Schmelzpunkt 187-190 °C.

c) Unter den Bedingungen des Beispiels 14 d werden 3 g 7-Hydroxy-3-methylindol-2-carbonsäureethylester und 2 g tert.-Butylaminoacetonitril-Hydrochlorid umgesetzt, aufbereitet und man erhält 3,6 g 4-tert.-Butylaminoacetyl-7-hydroxy-3-methylindol-2-carbonsäureethylester-Hydrochlorid vom Zersetzungspunkt.

d) Unter den Bedingungen des Beispiels 14 e werden 3 g 4-tert.-Butylaminoacetyl-7-hydroxy-3-methylindol-2-carbonsäureethylester-Hydrochlorid hydriert, aufbereitet und man erhält 1,9 g 4-(1-Hydroxy-2-tert.-butylaminoethyl)-7-hydroxy-3-methylindol-2-carbonsäureethylester-Hydrochlorid vom Zersetzungspunkt 153-156 °C.

Beispiel 18

Unter den Bedingungen des Beispiels 16 wird 1 g 4-(1-Hydroxy-2-tert.-butylaminoethyl)-7-hydroxy-3-methylindol-2-carbonsäureethylester-Hydrochlorid hydrolysiert, aufbereitet und man erhält 560 mg 4-(1-Hydroxy-2-tert.-butylaminoethyl)-7-hydroxy-3-methylindol-2-carbonsäure vom Zersetzungspunkt 230-231 °C.

Beispiel 19

a) Eine Lösung von 7-Benzyloxyindol-2-carbonsäureethylester in 600 ml Tetrahydrofuran und 200 ml Methanol wird mit 500 ml 2 n Natronlauge versetzt und drei Stunden bei Raumtemperatur gerührt. Dann säuert man die Reaktionsmischung unter Eiskühlung mit 10 %iger Salzsäure an und extrahiert zweimal mit Ethylacetat. Die vereinigten organischen Phasen werden getrocknet, mit Aktivkohle behandelt und

eingeengt. Der Rückstand wird aus Toluol umkristallisiert und man erhält 35 g 7-Benzyloxyindol-2-carbonsäure vom Schmelzpunkt 166-168 °C.

b) 5,3 g 7-Benzyloxyindol-2-carbonsäure werden in 80 ml Dichlormethan suspendiert und nacheinander mit 4,1 g Dicyclohexylcarbodiimid, 1,9 g Phenol und 300 mg 4-Pyrrolidinopyridin versetzt. Man rührt die Reaktionsmischung 18 Stunden lang bei Raumtemperatur, filtriert den erhaltenen Niederschlag ab und extrahiert das Filtrat zweimal mit Wasser, zweimal mit 5 %iger Essigsäure und noch zweimal mit Wasser. Die organische Phase wird getrocknet, mit Aktivkohle behandelt und eingeengt. Der Rückstand wird über eine Kieselgelsäule mit Chloroform chromatographiert und man erhält 5,5 g 7-Benzyloxyindol-2-carbonsäure-phenylester als viskoses Öl.

c) Unter den Bedingungen des Beispiels 14 c wird eine Lösung von 5,4 g 7-Benzyloxyindol-2-carbonsäure-phenylester in einem Gemisch aus 50 ml Methanol und 30 ml Tetrahydrofuran hydriert, aufbereitet und man erhält 3,5 g 7-Hydroxyindol-2-carbonsäure-phenylester vom Schmelzpunkt 156-157 °C.

d) Unter den Bedingungen des Beispiels 14 d werden 2,5 g 7-Hydroxyindol-2-carbonsäure-phenylester und 1,5 g tert.-Butylaminoacetonitril-Hydrochlorid umgesetzt, aufbereitet und man erhält 2,2 g 4-tert.-Butylaminoacetyl-7-hydroxy-indol-2-carbonsäure-phenylester-Hydrochlorid vom Zersetzungspunkt 276 °C.

e) Unter den Bedingungen des Beispiels 14 e werden 1,5 g 4-tert.-Butylaminoacetyl-7-hydroxyindol-2-carbonsäure-phenylester-Hydrochlorid in einer Mischung aus 40 ml Methanol und 40 ml Tetrahydrofuran hydriert, aufbereitet und man erhält 1,35 g 4-(2-tert.-Butylamino-1-hydroxyethyl)-7-hydroxyindol-2-carbonsäure-phenylester-Hydrochlorid vom Zersetzungspunkt 165-166 °C.

Beispiel 20

a) 10,6 g 7-Benzyloxyindol-2-carbonsäure werden in 100 ml Toluol suspendiert, mit 5,8 ml Thionylchlorid versetzt und zweieinhalb Stunden lang unter Rühren auf 100° erhitzt. Man läßt die Reaktionsmischung erkalten, engt sie zur Trockne ein, versetzt den Rückstand noch zweimal mit Toluol und engt jeweils im Vakuum zur Trockne ein. Das erhaltene Öl wird in 30 ml Toluol gelöst und unter Rühren bei Raumtemperatur in eine Lösung von 7,1 g Dimethylaminoethanol in 80 ml Toluol eingetropft. Nach einer Stunde wird die Reaktionsmischung zur Trockne eingeengt, der Rückstand in Wasser gegossen und mit Methylacetat extrahiert. Die organischen Phasen werden vereinigt, getrocknet, mit Aktivkohle behandelt und zur Trockne eingeengt. Der Rückstand wird aus Isopropanol umkristallisiert und man erhält 9,1 g 7-Benzyloxyindol-2-carbonsäure-2-dimethylaminoethylester vom Schmelzpunkt 113-115 °C.

b) Unter den Bedingungen des Beispiels 14 c werden 9 g 7-Benzyloxyindol-2-carbonsäure-2-dimethylaminoethylester in einer Mischung aus 50 ml Methanol und 50 ml Tetrahydrofuran hydriert, aufbereitet und man erhält 3,7 g 7-Hydroxyindol-2-carbonsäure-2-dimethylaminoethylester vom Schmelzpunkt 149-152 °C.

c) Unter den Bedingungen des Beispiels 14 d werden 2,5 g 7-Hydroxyindol-2-carbonsäure-dimethylaminoethylester und 1,5 g tert.-Butylaminoacetonitril-Hydrochlorid umgesetzt, aufbereitet und man erhält 2,6 g 4-tert.-Butylaminoacetyl-7-hydroxyindol-2-carbonsäure-2-dimethylaminoethylester-Hydrochlorid vom Zersetzungspunkt 266-268 °C.

d) Unter den Bedingungen des Beispiels 14 e werden 869 mg 4-tert.-Butylaminoacetyl-7-hydroxyindol-2-carbonsäure-2-dimethylaminoethylester-Hydrochlorid hydriert, aufbereitet und man erhält 510 mg 4-(2-tert.-Butylamino-1-hydroxyethyl)-7-hydroxyindol-2-carbonsäure-2-dimethylaminoethylester-Hydrochlorid vom Zersetzungspunkt 176 °C.

Beispiel 21

a) Zu einer Lösung von 5,37 g 7-Benzyloxyindol-2-carbonsäure in 20 ml Dimethylformamid werden 2,2 g Triethylamin und 3,6 g Brommethylacetat gegeben und die Mischung 5 Stunden bei Raumtemperatur gerührt. Die Reaktionsmischung wird in 150 ml Eiswasser gegeben, der Niederschlag abgesaugt, in 300 ml Chloroform gelöst und mit 10 %iger Natriumhydrogencarbonatlösung und Wasser gewaschen. Man trocknet die organische Phase, engt sie zur Trockne ein, kristallisiert den Rückstand aus Ethylacetat um und erhält 3,8 g 7-Benzyloxyindol-2-carbonsäure-acetoxymethylester vom Schmelzpunkt 170-171 °C.

b) Unter den Bedingungen des Beispiels 14 c werden 2,27 g 7-Benzyloxyindol-2-carbonsäure-acetoxymethylester in 40 ml Tetrahydrofuran hydriert, aufbereitet und man erhält 1,43 g 7-Hydroxyindol-2-carbonsäure-acetoxymethylester vom Schmelzpunkt 159-160 °C.

c) Unter den Bedingungen des Beispiels 14 d werden 2,5 g 7-Hydroxyindol-2-carbonsäure-acetoxymethylester mit 1,48 g tert.-Butylaminoacetonitril-Hydrochlorid umgesetzt, aufbereitet und man erhält 3,43 g 4-tert.-Butylaminoacetyl-7-hydroxyindol-2-carbonsäureacetoxymethylester-Hydrochlorid vom Zersetzungspunkt 261 °C.

d) Unter den Bedingungen des Beispiels 14 e werden 300 mg 4-tert.-Butylaminoacetyl-7-hydroxyindol-2-carbonsäureacetoxymethylester-Hydrochlorid hydriert, aufbereitet und man erhält 202 mg 4-(2-tert.-Butylamino-1-hydroxyethyl)-7-hydroxyindol-2-carbonsäureacetoxymethylester-Hydrochlorid vom Zersetzungspunkt 168 °C.

Beispiel 22

a) Eine Lösung von 12,3 g 3-(N-Benzyl-N-cyanomethylamino)-buttersäuremethylester in einer Mischung aus 100 ml Wasser und 25 ml 65 %iger Salpetersäure wird unter Rühren 2 Stunden lang auf 80° erhitzt. Man läßt die Reaktionsmischung erkalten, versetzt sie bis zur schwach sauren Reaktion mit festem Natriumacetat und extrahiert sie dreimal mit Diethylether. Die vereinigten organischen Phasen werden zweimal mit 10 %iger wässriger Natriumcarbonatlösung ausgeschüttelt, die vereinigten wässrigen Phasen mit Essigsäure angesäuert und dreimal mit Dichlormethan extrahiert.

Die vereinigten organischen Phasen werden getrocknet, mit Aktivkohle behandelt, zur Trockne eingeengt und der Rückstand mit Pentan verrieben. Man saugt die erhaltenen Kristalle ab, und erhält 7,8 g 3-(N-Benzyl-N-cyanomethylamino)-buttersäure vom Schmelzpunkt 71-73 °C.

b) Zu einer Lösung von 4,6 g 3-(N-Benzyl-N-cyanomethylamino)-buttersäure in 100 ml n-Butanol gibt man 0,5 ml konzentrierte Schwefelsäure und erhitzt die Mischung 4 Stunder lang am Wasserabscheider unter Rückfluß. Man läßt die Reaktionsmischung erkalten, engt sie zur Trockne ein und löst den Rückstand in Diethylether. Die etherische Lösung wird mit 10 %iger Natriumhydrogencarbonatlösung und gesättigter Kochsalzlösung gewaschen, getrocknet und zur Trockne eingeengt. Der ölige Rückstand wird im Kugelrohr bei 85-105 °C und 0,01 Torr destilliert und man erhält 1,17 g 3-(N-Benzyl-N-cyanomethylamino)-buttersäure-n-butylester als Öl.

c) Zu einer Suspension von 10,8 g 7-Benzyloxyindol-2-carbonsäure in 180 ml Toluol gibt man 5,8 ml Thionylchlorid und erhitzt die Mischung unter Rühren 2 Stunden lang auf 90°. Dann engt man das Reaktionsgemisch im Vakuum ein, versetzt den Rückstand mit 150 ml n-Butanol und erhitzt eine Stunde lang unter Rückfluß. Anschließend engt man die Mischung zur Trockne ein, kristallisiert den Rückstand aus Isopropanol um und erhält 7,4 g 7-Benzyloxyindol-2-carbonsäure-n-butylester vom Schmelzpunkt 77-78 °C.

d) Unter den Bedingungen des Beispiels 14 c wird eine Lösung von 5,8 g 7-Benzyloxyindol-2-carbonsäure-n-butylester in 200 ml Methanol hydriert, aufbereitet und man erhält 3,2 g 7-Hydroxyindol-2-carbonsäure-n-butylester vom Schmelzpunkt 150-151 °C.

e) Unter den Bedingungen des Beispiels 14 e werden 700 ml 7-Hydroxyindol-2-carbonsäure-n-butylester und 865 mg 3-(N-Benzyl-N-cyanomethylamino)-buttersäure-n-butylester umgesetzt, aufbereitet und man erhält 495 mg 4-(2-n-Butoxycarbonyl-1-methyl-ethylaminoacetyl)-7-hydroxyindol-2-carbonsäure-n-butylester-Hydrochlorid vom Zersetzungspunkt 200-203 °C.

f) Unter den Bedingungen des Beispiels 14 e werden 380 mg 4-(2-n-Butoxycarbonyl-1-methylethylaminoacetyl)-7-hydroxyindol-2-carbonsäure-n-butylester-Hydrochlorid in 10 ml Methanol und 5 ml Tetrahydrofuran hydriert, aufbereitet und man erhält 185 mg 4-[1-Hydroxy-2-(2-n-butoxycarbonyl-1-methylethylamino)-ethyl]-7-hydroxyindol-2-carbonsäure-n-butylester-Hydrochlorid vom Zersetzungspunkt 75-84 °C.

Beispiel 23

a) 48,8 g 4-[N-Benzyl-N-(2-methoxycarbonyl-1-methylethyl)-aminoacetyl]-7-hydroxyindol-2-carbonsäureethylester-Hydrochlorid 41,4 g Kaliumcarbonat und 25 ml Benzylbromid werden in 900 ml Aceton 6 Stunden lang unter Rückfluß erhitzt. Nach dem Erkalten wird die Reaktionsmischung zur Trockne eingeengt, der Rückstand in Ethylacetat und gesättiger Natriumhydrogencarbonatlösung aufgenommen die organische Phase abgetrennt und noch dreimal mit gesättigter Natriumhydrogencarbonatlösung extrahiert. Die organische Phase wird mit Wasser gewaschen, getrocknet, eingeengt und der Rückstand aus Ethylacetat/n-Hexan umkristallisiert. Man erhält 42,5 g 4-[N-Benzyl-N-(2-methoxycarbonyl-1-methylethyl)-aminoacetyl]-7-benzyloxyindol-2-carbonsäureethylester vom Schmelzpunkt 120-123 °C.

b) 38 g 4-[N-Benzyl-N-(2-methoxycarbonyl-1-methylethyl)-aminoacetyl]-7-benzyloxyindol-2-carbonsäureethylester werden in einer Mischung aus 1 Liter Tetrahydrofuran, 1 Liter Methanol und 350 ml 2-n-Natronlauge 4 Stunden lang bei Raumtemperatur gerührt. Anschließend säuert man die Reaktionsmischung mit 2-n-Salzsäure an, engt sie bis zur beginnenden Kristallisation ein, kühlt im Eisbad und saugt die erhaltenen Kristalle ab. Man erhält 25,8 g 4-[N-Benzyl-N-(2-carboxy-1-methylethyl)-aminoacetyl]-7-benzyloxyindol-2-carbonsäure-Hydrochlorid welches sich ab 150 °C zersetzt.

c) Unter den Bedingungen des Beispiels 21 a werden 5 g 4-[N-Benzyl-N-(2-carboxy-1-methylethyl)-aminoacetyl]-7-benzyloxyindol-2-carbonsäure-Hydrochlorid mit 1,6 g Chlormethylmethylether umgesetzt, aufbereitet und man erhält, nach Behandeln des Rohproduktes mit Cioxan/Essigsäure 4,2 g 4-[N-Benzyl-N-(2-methoxymethyloxycarbonyl-1-methylethyl)-aminoacetyl]-7-benzyloxyindol-2-carbonsäure-methoxymethyl-ester-Acetat vom Zersetzungspunkt 118-122 °C.

d) Unter den Bedingungen des Beispiels 14 e werden 3,5 g 4-[N-Benzyl-N-(2-methoxymethyloxy-carbonyl-1-methylethyl)-aminoacetyl]-7-benzyloxyindol-2-carbonsäuremethoxymethyl-ester-Acetat hydriert, aufbereitet und man erhält 2,1 g 4-[1-Hydroxy-2-(2-methoxymethylcarbonyl-1-methylethylamino)-ethyl]-7-hydroxyindol-2-carbonsäuremethoxymethylester-Acetat vom Zersetzungspunkt 110 °C.

Beispiel 24

a) Unter den Bedingungen des Beispiels 21 a werden 5,14 g 4-[N-Benzyl-N-(2-carboxy-1-methylethyl)-aminoacetyl]-7-benzyloxyindol-2-carbonsäure-Hydrochlorid mit 3,06 g Brommethylacetat umgesetzt, aufbereitet und man erhält nach Behandeln des Rohprodukts mit Dioxan/Essigsäure 2,80 g 4-[N-Benzyl-N-(2-acetoxymethyloxycarbonyl-1-methylethyl)-aminoacetyl]-7-benzyloxyindol-2-carbonsäureacetoxymethylester-Acetat vom Zersetzungspunkt 97-101 °C.

b) Unter den Bedingungen des Beispiels 14 e werden 2,50 g 4-[N-Benzyl-N-(2-acetoxymethyloxy-carbonyl-1-methylethyl)-aminoacetyl]-7-benzyloxyindol-2-carbonsäureacetoxymethylester-Acetat hydriert, aufbereitet und man erhält 1,10 g 4-[1-Hydroxy-2-(2-acetoxymethyloxycarbonyl-1-methylethylamino)-ethyl]-7-hydroxyindol-2-carbonsäureacetoxymethylester-Acetat vom Zersetzungspunkt 82 °C.

Beispiel 25

a) Unter den Bedingungen des Beispiels 21 a werden 5,14 g 4-[N-Benzyl-N-(2-carboxy-1-methylethyl)-aminoacetyl]-7-benzyloxyindol-2-carbonsäure-Hydrochlorid mit 3,00 g 2-Dimethylaminoethylchlorid-Hydrochlorid umgesetzt, aufbereitet und man erhält nach Behandeln des Rohprodukts mit Dioxan/Essigsäure 1,80 g 4-{N-Benzyl-N-[2-(2-dimethylaminoethyloxycarbonyl)-1-methylethyl]-aminoacetyl}-7-benzyloxyindol-2-carbonsäure-2-dimethylaminoethylester-Acetat vom Zersetzungspunkt 122-125 °C.

b) Unter den Bedingungen des Beispiels 14 e werden 1,50 g 4-{N-Benzyl-N-[2-(2-dimethylaminoethyloxycarbonyl)-1-methylethyl]-aminoacetyl}-7-benzyloxyindol-2-carbonsäure-2-dimethylaminoethylester-Acetat hydriert, aufbereitet und man erhält 0,40 g 4-{1-Hydroxy-2-[2-(2-dimethylaminoethyloxycarbonyl)-1-methylethylamino]-ethyl}-7-hydroxyindol-2-carbonsäure-2-dimethylaminoethylester-Acetat vom Zersetzungspunkt 102 °C.

Beispiel 26

a) Unter den Bedingungen des Beispiels 21 a werden 5,14 g 4-[N-Benzyl-N-(2-carboxy-1-methylethyl)-aminoacetyl]-7-benzyloxyindol-2-carbonsäure-Hydrochlorid mit 4,50 g 2-Morpholinoethylchlorid-Hydrochlorid umgesetzt, aufbereitet und man erhält nach Behandeln des Rohprodukts mit Dioxan/Essigsäure 2,20 g 4-N-Benzyl-N-[2-(2-Morpholinoethyloxycarbonyl)-1-methylethyl-aminoacetyl]-7-benzyloxyindol-2-carbonsäure-2-morpholinoethylester-Acetat vom Zersetzungspunkt 133-136 °C.

b) Unter den Bedingungen des Beispiels 14 e werden 2,00 g 4-N-Benzyl-N-[2-(2-morpholinoethyloxy-carbonyl)-1-methylethyl-aminoacetyl]-7-benzyloxyindol-2-carbonsäure-2-morpholinoethylester-Acetat hydriert, aufbereitet und man erhält 0,80 g 4{-1-Hydroxy-2-[2-(2-morpholinoethyloxycarbonyl)-1-methylethylamino]-ethyl}-7-hydroxyindol-2-carbonsäure-2-morpholinoethylester-Acetat vom Zersetzungspunkt 120 °C.

Beispiel 27

a) In eine Lösung von 7,6 ml Chloracetonitril in 100 ml Dioxan werden unter Eiskühlung 15 Minuten lang Chlorwasserstoff eingeleitet. Dann setzt man 53 g Aluminiumchlorid zu leitet unter Eiskühlung nochmals 15 Minuten lang Chlorwasserstoff ein und tropft unter weiterer Chlorwasserstoffeinleitung innerhalb von 30 Minuten eine Lösung von 7-Hydroxyindol-2-carbonsäureethylester in 200 ml Dioxan in

die Mischung. Man läßt die Reaktionsmischung zweieinhalb Stunden bei 0 °C stehen, gießt sie in 2 Liter Eiswasser und rührt anderthalb Stunden lang bei Raumtemperatur. Der ausgefallene Niederschlag wird abgesaugt, mit Wasser gewaschen, aus Methanol umkristallisiert und man erhält 22,2 g 4-Chloracetyl-7-hydroxyindol-2-carbonsäure-ethylester vom Schmelzpunkt 230-232 °C (Zersetzung).

b) 2,8 g 4-Chloracetyl-7-hydroxyindol-2-carbonsäure-ethylester werden mit 1,5 ml Allylamin und 15 ml absolutem Dimethylsulfoxid versetzt und 1 Stunde lang bei Raumtemperatur gerührt. Dann gibt man zur Reaktionsmischung 150 ml 2 n Salzsäure, saugt den sofort gebildeten Niederschlag ab, und läßt das Filtrat stehen. Aus diesem kristallisieren 1,5 g 4-Allylaminoacetyl-7-hydrxyindol-2-carbonsäure-ethylester-Hydrochlorid vom Schmelzpunkt 264-265° (Zersetzung).

c) 680 mg 4-Allylaminoacetyl-7-hydroxyindol-2-carbonsäure-ethylester-Hydrochlorid werden mit 100 ml Wasser und 50 ml Methanol versetzt und auf 40 °C erwärmt. Innerhalb von vier Stunden setzt man zu dieser Mischung portionsweise 4 g Natriumborhydrid zu, kühlt mit Eis, säuert mit 5 n Essigsäure pH 5 an und extrahiert zweimal mit Diethylether.

Die wässrige Phase wird mit Natriumhydrogencarbonat versetzt und dreimal mit n-Butanol-Ethylacetat (1 + 3) extrahiert. Man engt die organische Phase ein, löst den Rückstand in 25 ml 2-n-Essigsäure, extrahiert dreimal mit Diethylether, versetzt die wässrige Phase mit Natriumhydrogencarbonat und extrahiert dreimal mit Ethylacetat. Die organische Phase wird getrocknet, im Vakuum eingeengt und der Rückstand über eine Kieselgelsäule (Eluens : Toluol/Essigsäure/Methanol/Wasser (6 + 4 + 2 + 0,6) chromatographiert und man erhält nach Umkristallisation aus Methanol/Essigsäure/Diisopropylether 260 mg 7-Hydroxy-4-(1-hydroxy-2-allylaminoethyl)-indol-2-carbonsäure-ethylester-Acetat vom Schmelzpunkt 129-130 °C (Zersetzung).

## Beispiel 28

a) 3,4 g 4-Chloracetyl-7-hydroxyindol-2-carbonsäure-ethylester werden mit 4,2 g 1-(4-Benzyloxyphenyl)-propyl-2-amin-Hydrochlorid, 4,2 ml Triethylamin und 15 ml Dimethylsulfoxid versetzt und 4 Stunden lang bei Raumtemperatur gerührt. Dann versetzt man die Mischung mit 200 ml 2-n-Salzsäure und 100 ml Ethylacetat, saugt das Kristallisat ab und wäscht es mit 2-n-Salzsäure und Ethylacetat. Man erhält 1,8 g 4-[2-(4-Benzyloxyphenyl)-1-methylethylaminoacetyl]-7-hydroxyindol-2-carbonsäureethylester-Hydrochlorid vom Zersetzungspunkt 248° (aus Methanol/Ethylacetat).

b) 209 mg 4-[2-(4-Benzyloxyphenyl-1-methylethyl]-aminoacetyl-7-hydroxyindol-2-carbonsäureethylester-Hydrochlorid werden in 20 ml Methanol in Gegenwart von 42 mg 10 %iger Palladiumkohle 2,5 Stunden lang bei Raumtemperatur und Normaldruck hydriert. Man filtriert, engt das Filtrat im Vakuum ein kristallisiert den Rückstand aus Aceton/Diethylether um und erhält 115 mg 4-{1-Hydroxy-2-[2-(4-hydroxyphenyl)-1-methylethylamino]-ethyl}-indol-2-carbonsäureethylester-Hydrochlorid vom Zersetzungspunkt 110 °C.

## Beispiel 29

a) Unter den Bedingungen des Beispiels 28 werden 2,57 g 4-Chloracetyl-7-hydroxyindol-2-carbonsäureethylester mit 2,35 g 3-Benzylamino-3-methyl-buttersäureethylester umgesetzt, aufbereitet und man erhält 0,75 g 4-[N-Benzyl-N-(2-ethoxycarbonyl-1,1-dimethylethyl)-aminoacetyl]-7-hydroxyindol-2-carbonsäureethylester-Hydrochlorid vom Zersetzungspunkt 210-212 °C.

b) Unter den Bedingungen des Beispiels 14 e werden 4-[N-Benzyl-N-(2-ethoxycarbonyl-1,1-dimethylethyl)-aminoacetyl]-7-hydroxyindol-2-carbonsäureethylester hydriert, aufbereitet und man erhält 320 mg 4-[1-Hydroxy-(2-ethoxycarbonyl-1,1-dimethylethylamino)-ethyl]-7-hydroxyindol-2-carbonsäureethylester vom Schmelzpunkt 175-178 °C.

## Beispiel 30

a) 1,8 g 4-Chloracetyl-7-hydroxyindol-2-carbonsäureethylester und 1,8 g N,N'-Dibenzylhexamethylendiamin werden in 25 ml absolutem Dimethylsulfoxid 2 Stunden lang bei Raumtemperatur gerührt, mit Ethylacetat versetzt, das ausgefallene Kristallisat abgesaugt und die Mutterlauge eingeengt. Der Rückstand wird über eine Kieselgelsäule (Eluens: Methanol/Chloroform/Aceton 4 + 6 + 2) chromatographiert und aus Aceton, 1 n etherischer Salzsäure/Diethylether umkristallisiert und man erhält 775 mg N,N'-Hexamethylen-bis-[4-(N-benzylaminoacetyl)-7-hydroxyindol-2-carbonsäureethylester]-Dihydrochlorid vom Zersetzungspunkt 219-221 °C.

b) 687 mg N,N'-Hexamethylen-bis-[4-(N-benzylaminoacetyl)-7-hydroxyindol-2-carbonsäureethylester]-Dihydrochlorid in 80 ml Methanol werden mit 500 mg 10 %iger Palladiumkohle versetzt und

36 Stunden lang bei Raumtemperatur unter Normaldruck hydriert. Dann filtriert man den Katalysator ab, engt im Vakuum ein und kristallisiert den Rückstand aus Methanol/Ethylacetat um. Man erhält 300 mg N,N'-Hexamethylen-bis-[4-(2-amino-1-hydroxyethyl)-7-hydroxyindol-2-carbonsäureethylester]-Dihydrochlorid vom Zersetzungspunkt 142 °C.

Beispiel 31

a) In 300 ml Dioxan werden unter Eiskühlung 15 Minuten lang Chlorwasserstoff eingeleitet. Dann setzt man 33 g N-tert.-Butylaminoacetonitril-Hydrochlorid zu, leitet weitere 30 Minuten unter Eiskühlung Chlorwasserstoff ein, kühlt auf —30 °C, setzt 106 g Aluminiumchlorid zu, leitet weitere 30 Minuten lang unter Eiskühlung Chlorwasserstoff ein und versetzt mit 41 g 7-Hydroxy-indol-2-carbonsäureethylester in 300 ml Dioxan.

Die Reaktionsmischung wird unter Eiskühlung und Chlorwasserstoffeinleitung 2 Stunden lang und dann weitere 3 Stunden lang unter Eiskühlung gerührt. Man saugt den Niederschlag ab, wäscht ihn mit Dioxan und rührt ihn 3 Stunden lang mit 700 ml Wasser und 50 ml konzentrierter Salzsäure. Dann saugt man das Produkt ab, trocknet es, kristallisiert es aus Methanol/Diethylether um und erhält 58,9 g 4-(N-tert.-Butylaminoacetyl)-7-hydroxyindol-2-carbonsäure-ethylester-Hydrochlorid vom Zersetzungspunkt 271 °C.

b) 53,6 g 4-(N-tert.-Butylaminoacetyl)-7-hydroxyindol-2-carbonsäure-ethylester-Hydrochlorid werden mit 59,3 g Kaliumcarbonat, 63,6 ml Benzylbromid und 1 000 ml Aceton versetzt und 6 Stunden lang unter Rückfluß erhitzt. Dann engt man das Gemisch im Vakuum ein und versetzt den Rückstand mit Ethylacetat und gesättigter Natriumhydrogencarbonatlösung. Die organische Phase wird dreimal mit Natriumhydrogencarbonatlösung und einmal mit Wasser gewaschen, über Calciumsulfat getrocknet und eingeengt. Der Rückstand wird zweimal aus Ethylacetat/Hexan umkristallisiert und man erhält 47 g 7-Benzyloxy-4-(N-benzyl-N-tert.-butylaminoacetyl)-indol-2-carbonsäureethylester vom Schmelzpunkt 155-157 °C.

c) 10 g 7-Benzyloxy-4-(N-benzyl-N-tert.-butylaminoacetyl)-2-indol-carbonsäure-ethylester werden mit 300 ml Tetrahydrofuran, 300 ml Methanol und 100 ml 2-n-Natronlauge versetzt und vier Stunden lang bei Raumtemperatur gerührt. Dann setzt man 120 ml 2-n-Salzsäure zu und engt bis zur beginnenden Kristallisation ein. Das Kristallisat wird abgesaugt und die Mutterlauge abermals eingeengt. Das erhaltene Zweitkristallisat wird mit dem Erstkristallisat vereinigt, getrocknet, aus Methanol/Ethylacetat umkristallisiert und man erhält 8,8 g 7-Benzyl-oxy-4-(N-benzyl-N-tert.-butylaminoacetyl)-indol-2-carbonsäure-Hydrochlorid vom Zersetzungspunkt 158 °C.

d) 1,00 g 7-Benzyloxy-4-(N-benzyl-N-tert.-butylaminoacetyl)-2-indolcarbonsäure-Hydrochlorid 1,4 ml Triethylamin und 1,12 ml Trimethylessigsäurechlormethylester werden mit 15 ml absolutem Dimethylformamid versetzt und 16 Stunden lang bei Raumtemperatur gerührt. Dann engt man die Mischung ein, löst den Rückstand in Ethylacetat extrahiert dreimal mit Wasser, trocknet die organische Phase und engt sie ein. Der Rückstand wird über eine Aluminiumoxidsäule (Aktivitätsstufe II ; Eluens : Cyclohexan/Ethylacetat 4 + 1) chromatographiert, aus Diisopropylether/etherischer Salzsäure umkristallisiert und man erhält 0,7 g 7-Benzyloxy-4-(N-benzyl-N-tert.-butyl-aminoacetyl)-2-indolcarbonsäure-trimethylacetoxymethyl-ester-Hydrochlorid vom Zersetzungspunkt 184-185 °C.

e) 609 mg 7-Benzyloxy-4-(N-benzyl-N-tert.-butylaminoacetyl)-2-indolcarbonsäure-trimethylacetoxymethylester-Hydrochlorid werden mit 30 ml absolutem Tetrahydrofuran, 3 ml Methanol und 0,5 ml Essigsäure versetzt und in Gegenwart von 100 mg 10 %iger Palladiumkohle 10 Stunden lang bei Raumtemperatur unter Normaldruck hydriert.

Dann engt man die Mischung ein, kristallisiert den Rückstand aus Methanol/Diethylether um und erhält 300 mg 7-Hydroxy-4-(1-hydroxy-2-tert.-butylaminoethyl)-indol-2-carbonsäure-trimethylacetoxymethylester vom Zersetzungspunkt 173-175 °C.

Beispiel 32

a) 1,00 g 7-Benzyloxy-4-(N-benzyl-N-tert.-butylaminoacetyl)-2-indolcarbonsäure-Hydrochlorid, 0,6 ml Chlormethylmethylether, 1,4 ml Triethylamin werden in 20 ml absolutem Dimethylformamid gelöst und eine Stunde bei 0 °C und 16 Stunden bei Raumtemperatur gerührt.

Dann engt man die Mischung im Vakuum ein, versetzt den Rückstand mit Ethylacetat und Wasser, saugt das Kristallisat ab und erhält 680 mg 7-Benzyloxy-4-(N-benzyl-N-tert.-butylaminoacetyl)-2-indolcarbonsäure-methoxymethylester vom Schmelzpunkt 178-179 °C (aus Tetrahydrofuran/Diisopropylether).

b) 617 mg 7-Benzyloxy-4-(N-benzyl-N-tert-butylaminoacetyl)-2-indolcarbonsäure-methoxymethylester werden mit 10 ml Methanol, 40 ml absolutem Tetrahydrofuran und 0,3 ml Essigsäure versetzt und

wie im Beispiel 29 e hydriert und aufbereitet. Man erhält 450 mg 7-Hydroxy-4-(1-hydroxy-2-tert.-butylaminoethyl)-indol-2-carbonsäure-methoxy-methylester-Acetat vom Zersetzungspunkt 165-170 °C.

Beispiel 33

a) 1,00 g 7-Benzyloxy-4-(N-benzyl-N-tert.-butylaminoacetyl)-2-indolcarbonsäure-Hydrochlorid werden in 4 ml absolutem Dimethylformamid und 8 ml absolutem Tetrahydrofuran versetzt, auf — 10 °C gekühlt und mit 0,52 ml N-Ethylmorpholin und fünf Minuten später mit 0,28 ml Chlorameisensäureisobutylester versetzt. Man läßt 30 Minuten bei — 5° stehen, gibt 376 mg Aminoessigsäuremethylester-Hydrochlorid, 0,39 ml Triethylamin — gelöst in 4 ml absolutem Dimethylformamid und 6 ml absolutem Tetrahydrofuran — hinzu, rührt 16 Stunden bei Raumtemperatur, engt ein, nimmt den Rückstand in Ethylacetat/Wasser auf, säuert die wässrige Phase mit 1 n-Salzsäure an und extrahiert nochmals mit Ethylacetat. Die vereinigten organischen Phasen werden getrocknet, eingeengt, über eine Aluminiumoxidsäule (Aktivitätsstufe II, Eluens : Methanol/Chloroform 1 + 4) chromatographiert und man erhält 600 mg N-[7-Benzyloxy-4-(N-benzyl-N-tert.-butylaminoacetyl)-2-indolylcarbonyl]-aminoessigsäuremethylester vom Schmelzpunkt 151,5-152,5 °C (aus Methanol/Diisopropylether).

b) 300 mg N-[7-Benzyloxy-4-(N-benzyl-N-tert.-butylaminoacetyl)-2-indolylcarbonyl]-aminoessigsäuremethylester werden mit 10 ml Methanol, 20 ml absolutem Tetrahydrofuran und 0,16 ml Essigsäure versetzt und wie im Beispiel 29 e beschrieben hydriert und aufbereitet. Man erhält 115 mg N-[7-Hydroxy-4-(1-hydroxy-2-tert.-butylaminoethyl)-2-indolylcarbonyl]-aminoessigsäuremethyl-ester-Acetat vom Zersetzungspunkt 140 °C.

Beispiel 34

a) 4 g 7-Benzyloxy-4-(N-benzyl-N-tert.-butylaminoacetyl)-2-indolcarbonsäureethylester werden mit 75 ml absolutem Tetrahydrofuran und 150 ml Diethylether versetzt und 9 Stunden lang unter Eiskühlung mit 1,2 g Lithiumaluminiumhydrid gerührt. Dann gitt man zur Reaktionsmischung wenig Wasser, saugt den Niederschlag ab, wäscht ihn mit Ethylacetat/Diethylether, engt die vereinigten Filtrate ein kristallisiert den Rückstand aus Diisopropylether um und erhält 3,4 g 2-(N-Benzyl-N-tert.-butyl-amino)-1-(7-benzyloxy-2-hydroxymethylindol-4-yl)-ethanol vom Schmelzpunkt 135,5-136,5 °C.

b) 687 mg 2-(N-Benzyl-N-tert.-butylamino)-1-(7-benzyloxy-2-hydroxymethylindol-4-yl)-ethanol werden mit 10 ml absolutem Tetrahydrofuran und 0,6 ml Essigsäureanhydrid 18 Stunden lang bei Raumtemperatur gerührt. Dann engt man das Gemisch ein, nimmt den Rückstand in Ethylacetat auf, wäscht mit gesättigter Natriumhydrogencarbonatlösung, trocknet die organische Phase und engt sie ein. Der Rückstand wird über eine Aluminiumoxidsäule (Aktivitätsstufe II, Eluens : Cyclohexan/Ethylacetat 1,5 + 1) chromatographiert aus Diisopropylether/Hexan umkristallisiert und man erhält 515 mg Essigsäure-{7-Benzyloxy-4-[2-(N-benzyl-N-tert.-butylamino)-1-hydroxyethyl]-indol-2-yl-methyl} ester vom Schmelzpunkt 133-134 °C.

c) 500 mg der erhaltenen Verbindung werden in 20 ml absolutem Tetrahydrofuran und 5 ml Essigsäure 45 Minuten lang in Gegenwart von 40 mg 10 %iger Palladiumkohle hydriert. Man filtriert, engt das Filtrat ein, verreibt den Rückstand zweimal mit absolutem Diethylether und zweimal mit Aceton/Diethylether und erhält 255 mg Essigsäure-[4-(2-tert.-butylamino-1-hydroxyethyl)-7-hydroxyindol-2-yl-methyl]-ester-Acetat vom Zersetzungspunkt 162-163 °C.

Beispiel 35

229 mg 2-(N-Benzyl-N-tert.-butylamino)-1-(7-benzyloxy-2-hydroxymethylindol-4-yl)-ethanol werden in 10 ml Methanol 10 Minuten lang in Gegenwart von 40 mg 10 %iger Palladiumkohle hydriert, filtriert, das Filtrat eingeengt aus Ethanol/Diisopropylether umkristallisiert und man erhält 95 mg 2-(N-tert.-butylamino)-1-(7-hydroxy-2-hydroxymethylindol-4-yl)-ethanol vom Schmelzpunkt 150 °C (Zersetzung).

Beispiel 36

550 Essigsäure-{7-Benzyloxy-4-[2-(N-benzyl-N-tert.-butylamino-1-hydroxyethyl]-indol-2-yl}-methylester werden in 20 ml Methanol 30 Minuten lang in Gegenwart von 75 mg 10 %iger Palladiumkohle hydriert. Man filtriert, engt das Filtrat ein und kristallisiert den Rückstand aus Ethanol/Diisopropylether um. Man erhält so 285 mg 2-(N-tert.-Butyl-amino)-1-(7-hydroxy-2-methylindol-4-yl)-ethanol-Acetat vom Zersetzungspunkt 177-178 °C.

Beispiel 37

a) Unter den Bedingungen des Beispiels 28 werden 2,57 g 4-Chloracetyl-7-hydroxyindol-2-carbonsäureethylester mit 1,93 g D-L-3-Aminobuttersäurebenzylester umgesetzt, aufbereitet und man erhält 510

mg 4-[N-(2-Benzyloxy-carbonyl-1-methylethyl)-aminoacetyl]-7-hydroxyindol-2-carbonsäureethylester vom Zersetzungspunkt 165-169 °C.

b) Unter den Bedingungen des Beispiels 14 e werden 300 mg 4-[N-(2-Benzyloxycarbonyl-1-methylethyl)-aminoacetyl]-7-hydroxyindol-2-carbonsäureethylester-Hydrochlorid hydriert, aufbereitet und man erhält 110 mg 4-[1-Hydroxy-2-carboxy-1-methylethylamino)-ethyl]-7-hydroxy-indol-2-carbonsäureethylester-Hydrochlorid vom Zersetzungspunkt 176-179 °C.

Beispiel 38

a) Unter den Bedingungen des Beispiels 14 d werden 1,8 g 7-Hydroxyindol-2-carbonsäure mit 2,5 g 3-(N-Benzyl-N-cyanomethylamino)-buttersäure-methylester umgesetzt, aufbereitet und man erhält 1,4 g 4-[N-Benzyl-N-(2-methoxycarbonyl-1-methylethyl)-aminoacetyl]-7-hydroxyindol-2-carbonsäure-Hydrochlorid vom Zersetzungspunkt 245-250 °C.

b) Unter den Bedingungen des Beispiels 14 e werden 1,1 g 4-[N-Benzyl-N-(2-methoxycarbonyl-1-methylethyl)-amino-acetyl]-7-hydroxyindol-2-carbonsäure-Hydrochlorid hydriert, aufbereitet und man erhält 420 mg 4-[1-Hydroxy-2-(2-methoxy-carbonyl-1-1-methyethylamino)-ethyl]-7-hydroxyindol-2-carbonsäure-Hydrochlorid, das sich ab 120° zersetzt.

Beispiel 39

a) In eine Lösung von 19 g 7-Hydroxyindol-2-carbonsäure-ethylester und 5 g s-Triazin in 300 ml Tetrahydrofuran wird unter Eiskühlung 8 Stunden lang Chlorwasserstoff eingeleitet. Dann engt man die Reaktionsmischung zur Trockne ein, versetzt sie mit 200 ml Wasser und 200 ml Ethylacetat und rührt sie drei Stunden lang bei Raumtemperatur. Die organische Phase wird abgetrennt, die wässrige Phase noch zweimal mit Ethylacetat extrahiert, die organischen Phasen vereinigt und zur Trockne eingeengt. Der Rückstand wird mit 200 ml Ethylacetat ausgekocht, das erhaltene Kristallisat abgesaugt und man erhält 5,8 g 4-Formyl-7-hydroxyindol-2-carbonsäureethylester vom Schmelzpunkt 210-225 °C.

b) 2,33 g 4-Formyl-7-hydroxyindol-2-carbonsäure-ethylester werden mit 1,8 ml Benzylbromid, 1 g Kaliumcarbonat und 100 ml Aceton versetzt und 4 Stunden lang unter Rückfluß erhitzt. Dann engt man die Reaktionsmischung zur Trockne ein, versetzt mit Wasser und extrahiert mit Ethylacetat. Die organische Phase wird zweimal mit gesättigter Natriumhydrogencarbonatlösung gewaschen, über Natriumsulfat getrocknet, eingeengt und aus Ethylacetat/Hexan umkristallisiert. Man erhält so 1,8 g 7-Benzyloxy-4-formylindol-2-carbonsäureethylester vom Schmelzpunkt 131-132 °C.

c) 2,26 g 7-Benzyloxy-4-formylindol-2-carbonsäure-ethylester werden in einer Mischung aus 15 ml 1-n-Natronlauge und 15 ml Ethanol 1 Stunde lang auf 100° erhitzt. Dann destilliert man das Ethanol im Vakuum ab, säuert mit 10 %iger Salzsäure an und saugt den Niederschlag ab. Dieser wird aus Eisessig umkristallisiert und man erhält 2,22 g 7-Benzyloxy-4-formylindol-2-carbonsäure vom Schmelzpunkt 207 °C.

d) Man erhitzt 500 mg Kupferpulver in 30 ml Chinolin auf 160° und setzt dem Gemisch 2 g 7-Benzyloxy-4-formylindol-2-carbonsäure zu. Die Mischung wird innerhalb von 15 Minuten auf 220-230° erhitzt und 25 Minuten lang bei dieser Temperatur gehalten. Man läßt die Reaktionsmischung erkalten, gießt sie in 150 ml 10 %ige Salzsäure, filtriert und extrahiert das Filtrat dreimal mit je 100 ml Ethylacetat. Die vereinigten organischen Phasen werden getrocknet, eingeengt und der Rückstand über eine Kieselgelsäule mit Chloroform als Eluens chromatographiert. Man erhält 780 mg 7-Benzyloxy-4-formylindol vom Schmelzpunkt 141-142 °C (aus Methanol).

e) 500 mg 7-Benzyloxy-4-formylindol werden mit 50 mg 4-Di-methylaminopyridin, 0,7 ml Triethylamin, 0,5 ml Essigsäure-anhydrid und 10 ml Dichlormethan versetzt und 24 Stunden lang bei Raumtemperatur gerührt. Dann verdünnt man die Reaktionsmischung mit 50 ml Dichlormethan, wäscht mit konzentrierter Natriumhydrogencarbonatlösung, trocknet die organische Phase und engt sie ein. Der Rückstand wird aus Isopropanol umkristallisiert und man erhält 400 mg 1-Acetyl-7-benzyloxy-4-formylindol vom Schmelzpunkt 77-78 °C.

f) Eine Lösung von 130 mg 1-Acetyl-7-benzyloxy-4-formylindol in 5 ml Dimethylformamid wird mit 100 mg Trimethylsulfoniumjodid und 45 mg Natriumhydrid (80 %ig in Öl) versetzt und 20 Stunden lang bei Raumtemperatur gerührt. Dann versetzt man die Reaktionsmischung mit 50 ml Wasser, extrahiert dreimal mit Ethylacetat, wäscht die organische Phase dreimal mit gesättigter Kochsalzlösung trocknet sie und engt sie ein.

Das erhaltene ölige Produkt wird in 5 ml tert.-Butylamin gelöst und 4 Stunden lang unter Rückfluß erhitzt. Dann engt man die Mischung zur Trockne ein, kristallisiert aus Ethylacetat/Hexan um und erhält 90 mg 7-Benzyloxy-4-(1-hydroxy-2-tert.-butylaminoethyl)-indol das sich ab 80° zersetzt.

g) Unter den Bedingungen des Beispiels 14 e werden 800 mg 7-Benzyloxyindol-4-(1-hydroxy-2-tert.-butylaminoethyl)-indol hydriert, aufbereitet und man erhält 40 mg 4-(1-Hydroxy-2-tert.-butylaminoethyl)-7-hydroxyindol das sich ab 100° zersetzt.

Beispiel 40

a) 1 g 7-Benzyloxy-4-(N-benzyl-N-tert.-butylaminoacetyl)-indol-2-carbonsäure-Hydrochlorid werden mit 650 mg N-(2-hydroxyethyl)-morpholin, 366 mg 4-Dimethylaminopyridin, 450 mg N,N'-Dicyclohexylcarbodiimid, 5 ml absolutem Dimethylformamid und 15 ml absolutem Tetrahydrofuran versetzt und eine Stunde lang bei 0 °C und weitere 16 Stunden bei Raumtemperatur gerührt. Man filtriert die Reaktionsmischung, engt das Filtrat ein, nimmt den Rückstand in Ethylacetat auf, wäscht viermal mit Wasser, trocknet die organische Phase und engt sie ein. Der Rückstand wird über eine Alumminiumoxidsäule (Aktivitätsstufe II, Eluens : Ethylacetat/Cyclohexan 2 + 1) chromatographiert und man erhält 440 mg 7-Benzyloxy-4-(N-benzyl-N-butylaminoacetyl)-indol-2-carbonsäure-2-(morpholino)-ethylester vom Schmelzpunkt 149-150 °C (aus Ethylacetat/Diisopropylether).

b) 408 mg 7-Benzyloxy-4-(N-benzyl-N-tert.-butylaminoacetal)-indol-2-carbonsäure-2-(morpholino)-ethylester werden unter den Bedingungen des Beispiels 14 e hydriert, aufbereitet und man erhält 250 mg 7-Hydroxy-4-(1-hydroxy-2-tert.-butyl-aminoethyl)-indol-2-carbonsäure-2-(morpholino)-ethylester-monoacetat vom Zersetzungspunkt 177-177,5 °C (aus Methanol/Diethylether).

**Patentansprüche**

1. Indol-Derivate der allgemeinen Formel I

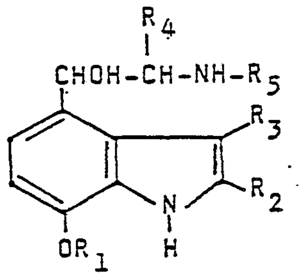

(I)

worin

$R_1$ ein Wasserstoffatom oder einen Methylrest und

$R_2$ ein Wasserstoffatom, einen bis zu 4 Kohlenstoffatome enthaltenden Alkylrest eine gegebenenfalls mit einer 1 bis 6 Kohlenstoffatomen enthaltenden Alkancarbonsäure veresterte Hydroxymethylgruppe oder eine Carboxylgruppe, eine Phenoxycarbonylgruppe oder eine gegebenenfalls durch ein Sauerstoffatom, ein Stickstoffatom oder eine Carboxygruppe unterbrochene Alkoxycarbonylgruppe, Cycloalkylalkoxycarbonylgruppe oder Alkylaminocarbonylgruppe mit maximal 8 Kohlenstoffatomen darstellen, und worin

$R_3$ ein Wasserstoffatom, oder eine Methylgruppe

$R_4$ ein Wasserstoffatom oder einen 1 bis 6 Kohlenstoffatome enthaltender Alkylrest bedeutet und

$R_5$ ein Wasserstoffatom, eine gegebenenfalls durch Hydroxygruppen, 1 bis 4 Kohlenstoffatome enthaltende Alkyloxygruppen, 1 bis 4 Kohlenstoffatome enthaltende Alkylgruppen oder Halogenatome substituierte Phenylalkylgruppe, mit 1 bis 6 Kohlenstoffatomen im Alkylteil, einen Allylrest, einen Alkylrest oder Cycloalkylrest mit maximum 6 Kohlenstoffatomen oder eine mit einer gegebenenfalls durch ein Sauerstoffatom, ein Stickstoffatom oder eine Carboxygruppe unterbrochenen Alkoxycarbonylgruppe oder Cycloalkylalkoxycarbonylgruppe substituierten Alkylgruppe mit insgesamt maximal 10 Kohlenstoffatomen, bedeutet oder eine zwei Indol-Reste der allgemeinen Formel I a

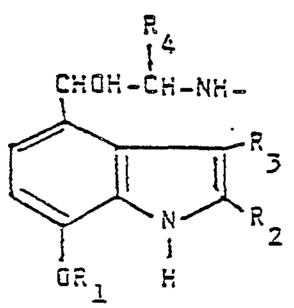

(Ia)

worin $R_1$, $R_2$, $R_3$ und $R_4$ die obengenannte Bedeutung besitzen, verbindende bis zu 8 Kohlenstoffatome

enthaltende Polymethylengruppe darstellt, sowie deren Salze von physiologisch unbedenklichen Säuren und gegebenenfalls deren Alkalimetallsalze und Erdalkalimetallsalze.

2. 4-(2-Amino-1-hydroxy-äthyl)-7-hydroxy-indol-2-carbonsäure-methylester und dessen Hydrochlorid.

3. 4-(1-Hydroxy-2-methylamino-äthyl)-7-hydroxy-indol-2-carbonsäure-methylester und dessen Hydrochlorid.

4. 4-(1-Hydroxy-2-isopropylamino-äthyl)-7-hydroxy-indol-2-carbonsäure-methylester und dessen Hydrochlorid.

5. 4-(1-Hydroxy-2-isopropylamino-äthyl)-7-hydroxy-indol-2-carbonsäure.

6. 4-(2-tert.-Butylamino-1-hydroxy-äthyl)-7-hydroxy-indol-2-carbonsäure-methylester und dessen Hydrochlorid.

7. 4-(2-tert.-Butylamino-1-hydroxy-äthyl)-7-hydroxy-indol-2-carbonsäure.

8. 4-(2-tert.-Butylamino-1-hydroxy-äthyl)-7-hydroxy-indol-2-carbonsäure-äthylester und dessen Hydrochlorid.

9. 4-(2-tert.-Butylamino-1-hydroxy-äthyl)-7-hydroxy-indol-2-carbonsäure-n-butylester und dessen Hydrochlorid.

10. 4-(2-tert.-Butylamino-1-hydroxy-äthyl)-7-hydroxy-indol-2-carbonsäure-isobutylester und dessen Hydrochlorid.

11. 4-(2-tert.-Butylamino-1-hydroxy-äthyl)-7-methoxy-indol-2-carbonsäure-methylester und dessen Hydrochlorid.

12. 4-[1-Hydroxy-2-(2-methoxycarbonyl-1-methyl-äthylamino)-äthyl]-7-hydroxyindol-2-carbonsäure-äthylester und dessen Hydrochlorid.

13. 4-(2-tert.-Butylamino-1-hydroxypropyl)-7-hydroxyindol-2-carbonsäure-äthylester und dessen Hydrochlorid.

14. 4-(2-Cyclohexylamino-1-hydroxyäthyl)-7-hydroxyindol-2-carbonsäure-ethylester-hydrochlorid und dessen Hydrochlorid.

15. 4-(1-Cyclopropylamino-2-hydroxyethyl)-7-hydroxyindol-2-carbonsäure-ethylester.

16. 4-{1-Hydroxy-2-[2-(4-methoxyphenyl)-1-methylethylamino]-ethyl}-7-hydroxyindol-2-carbonsäure-methylester.

17. 4-{1-Hydroxy-2-[2-(4-methoxyphenyl)-1-methylethylamino]-ethyl}-7-hydroxyindol-2-carbonsäure.

18. 4-(1-Hydroxy-2-tert.-butylaminoethyl)-7-hydroxy-3-methylindol-2-carbonsäure-ethylester.

19. 4-(1-Hydroxy-2-tert.-butylaminoethyl)-7-hydroxy-3-methylindol-2-carbonsäure.

20. 4-(2-tert.-Butylamino-1-hydroxyethyl)-7-hydroxyindol-2-carbonsäure-phenylester.

21. 4-(2-tert.-Butylamino-1-hydroxyethyl)-7-hydroxyindol-2-carbonsäure-2-dimethylaminoethylester.

22. 4-(2-tert.-Butylamino-1-hydroxyethyl)-7-hydroxyindol-2-carbonsäure-acetoxymethylester.

23. 4-[1-Hydroxy-2-(2-n-butoxycarbonyl-1-methylethylamino)-ethyl]-7-hydroxyindol-2-carbonsäure-n-butylester.

24. 4-[1-Hydroxy-2-(2-methoxymethyloxycarbonyl-1-methylethylamino)-ethyl]-7-hydroxyindol-2-carbonsäure-methoxymethyl-ester.

25. 4-[1-Hydroxy-2-(2-acetoxymethyloxycarbonyl-1-methyl-ethylamino)-ethyl]-7-hydroxyindol-2-carbonsäure-acetoxymethylester.

26. 4-{1-Hydroxy-2-[2-(2-diemetylaminoacetoxycarbonyl)-1-methylethylamino]-ethyl}-7-hydroxyindol-2-carbonsäure-2-dimethylaminoethylester.

27. 4-{1-Hydroxy-2-[2-(2-morpholinoethyloxycarbonyl)-1-methylethylamino]-ethyl}-7-hydroxyindol-2-carbonsäure-2-morpholino-ethylester.

28. 7-Hydroxy-4-(1-hydroxy-2-allylaminoethyl)-indol-2-carbonsäure-ethylester.

29. 4-{1-Hydroxy-2-[2-(4-hydroxyphenyl)-1-methylethylamino]-ethyl}-indol-2-carbonsäure-ethylester.

30. 4-[1-Hydroxy-(2-ethoxycarbonyl-1,1-dimethylethylamino)-ethyl]-7-hydroxyindol-2-carbonsäure-ethylester.

31. N,N'-Hexamethylen-bis-[4-(2-amino-1-hydroxyethyl)-7-hydroxyindol-2-carbonsäure]-ethylester.

32. 7-Hydroxy-4-(1-hydroxy-2-tert.-butylaminoethyl)-indol-2-carbonsäure-trimethylacetoxymethylester.

33. 7-Hydroxy-4-(1-hydroxy-2-tert.-butylaminoethyl)-indol-2-carbonsäure-methoxymethylester.

34. N-[7-Hydroxy-4-(1-hydroxy-2-tert.-butylaminoethyl-2-indolcarbonyl]-aminoessigsäure-methylester.

35. Essigsäure-[4-(2-tert.-butylamino-1-hydroxyethyl)-7-hydroxyindol-2-yl-methyl]-ester.

36. 2-(N-tert.-Butylamino)-1-(7-hydroxy-2-hydroxymethyl-indol-4-yl)-ethanol.

37. 2-(N-tert.-Butylamino)-1-(7-hydroxy-2-methylindol-4-yl)-ethanol.

38. 4-(1-Hydroxy-2-tert.-butylaminoethyl)-7-hydroxyindol.

39. 7-Hydroxy-4-(1-hydroxy-2-tert.-buylaminoethyl)-indol-2-carbonsäure-2-(morpholino)-ethylester.

40. Pharmazeutische Präparate gekennzeichnet durch einen Gehalt an Indol-Derivaten gemäß Anspruch 1 bis 39.

41. Verfahren zur Herstellung von Indol-Derivaten der allgemeinen Formel I gemäß Patentanspruch 1, dadurch gekennzeichnet, daß man in an sich bekannter Weise

a) ein Indol-Derivat der allgemeinen Formel II

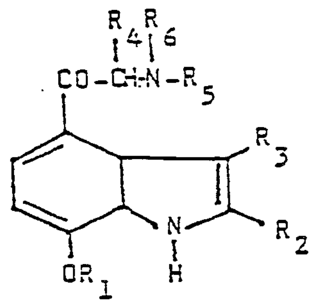

(II)

worin $R_1$, $R_2$, $R_3$, $R_4$ und $R_5$ die in Patentanspruch 1 genannte Bedeutung besitzen und $R_6$ ein Wasserstoffatom oder einen Benzylrest darstellt, gegebenenfalls unter gleichzeitiger Abspaltung der Benzylgruppen reduziert, oder

b) ein Indol-Derivat der allgemeinen Formel I mit $R_1$ in der Bedeutung einer Benzylgruppe hydrierend spaltet und gewünschtenfalls vorhandene Estergrüppen verseift, oder freie Säuren verestert und gewünschtenfalls die erhaltenen Verbindungen in ihre Salze überführt.

## Claims

1. Indole derivatives of the general formula I

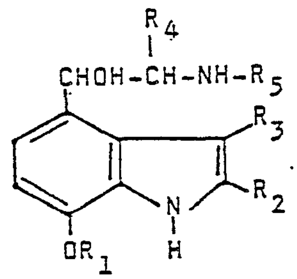

(I)

wherein

$R_1$ is hydrogen or methyl and

$R_2$ is hydrogen, alkyl containing up to 4 carbon atoms, hydroxymethyl, optionally esterified by an alkanoyl group containing 1 to 6 carbon atoms, a carboxy group, a phenoxycarbonyl group or an alkoxycarbonyl, cycloalkoxycarbonyl or alkylaminocarbonyl group, with a maximum of 8 carbon atoms, optionally interrupted by oxygen, nitrogen or a carboxy group, and wherein

$R_3$ is hydrogen or methyl,

$R_4$ is hydrogen or alkyl of 1 to 6 carbon atoms and

$R_5$ is hydrogen, a phenylalkyl group, optionally substituted by hydroxy, an alkoxy group containing 1 to 4 carbon atoms, an alkyl group containing 1 to 4 carbon atoms or halogen, an alkyl group with a maximum of 10 carbon atoms substituted by an alkoxycarbonyl or cycloalkylalkoxycarbonyl group which themselves may be optionally interrupted by oxygen, nitrogen or carbonyl, or a two indole groups of general formula Ia

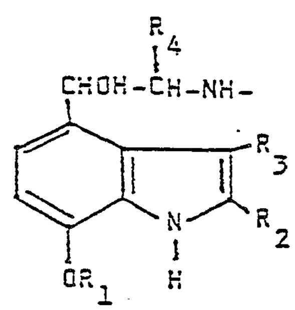

(Ia)

wherein $R_1$, $R_2$, $R_3$ and $R_4$ have the meanings given above, are joined by a polymethylene group containing up to 8 carbon atoms, as well as salts of these compounds with physiologically harmless acids and optionally alkali metal and alkaline earth metal salts of these compounds.

2. 4-(2-Amino-1-hydroxyethyl)-7-hydroxyindole-2-carboxylic acid methyl ester and its hydrochloride.

3. 4-(1-Hydroxy-2-methylaminoethyl)-7-hydroxyindole-2-carboxylic acid methyl ester and its hydrochloride.

4. 4-(1-Hydroxy-2-isopropylaminoethyl)-7-hydroxyindole-2-carboxylic acid methyl ester and its hydrochloride.

5. 4-(1-Hydroxy-2-isopropylaminoethyl)-7-hydroxyindole-2-carboxylic acid.

6. 4-(2-tert.-Butylamino-1-hydroxyethyl)-7-hydroxyindole-2-carboxylic acid methyl ester and its hydrochloride.

7. 4-(2-tert.-Butylamino-1-hydroxyethyl)-7-hydroxyindole-2-carboxylic acid.

8. 4-(2-tert.-Butylamino-1-hydroxyethyl)-7-hydroxyindole-2-carboxylic acid ethyl ester and its hydrochloride.

9. 4-(2-tert.-Butylamino-1-hydroxyethyl)-7-hydroxyindole-2-carboxylic acid n-butyl ester and its hydrochloride.

10. 4-(2-tert.-Butylamino-1-hydroxyethyl)-7-hydroxyindole-2-carboxylic acid isobutyl ester and its hydrochloride.

11. 4-(2-tert.-Butylamino-1-hydroxyethyl)-7-methoxyindole-2-carboxylic acid methyl ester and its hydrochloride.

12. 4-[1-Hydroxy-2-(2-methoxycarbonyl-1-methylethylamino)-ethyl]-7-hydroxyindole-2-carboxylic acid ethyl ester and its hydrochloride.

13. 4-(2-tert.-Butylamino-1-hydroxypropyl)-7-hydroxyindole-2-carboxylic acid ethyl ester and its hydrochloride.

14. 4-(2-Cyclohexylamino-1-hydroxyethyl)-7-hydroxyindole-2-carboxylic acid ethyl ester hydrochloride and its hydrochloride.

15. 4-(1-Cyclopropylamino-2-hydroxyethyl)-7-hydroxyindole-2-carboxylic acid ethyl ester.

16. 4-1-Hydroxy-2-[2-(4-methoxyphenyl)-1-methylethylamino]-ethyl-7-hydroxyindole-2-carboxylic acid methyl ester.

17. 4-1-Hydroxy-2-[2-(4-methoxyphenyl)-1-methylethylamino]-ethyl-7-hydroxyindole-2-carboxylic acid.

18. 4-(1-Hydroxy-2-tert.-butylaminoethyl)-7-hydroxy-3-methylindole-2-carboxylic acid ethyl ester.

19. 4-(1-Hydroxy-2-tert.-butylaminoethyl)-7-hydroxy-3-methylindole-2-carboxylic acid.

20. 4-(2-tert.-Butylamino-1-hydroxyethyl)-7-hydroxyindole-2-carboxylic acid phenyl ester.

21. 4-(2-tert.-Butylamino-1-hydroxyethyl)-7-hydroxyindole-2-carboxylic acid dimethylaminoethyl ester.

22. 4-(2-tert.-Butylamino-1-hydroxyethyl)-7-hydroxyindole-2-carboxylic acid acetoxymethyl ester.

23. 4-[1-Hydroxy-2-(2-n-butoxycarbonyl-1-methylethylamino)-ethyl]-7-hydroxyindole-2-carboxylic acid n-butyl ester.

24. 4-[1-Hydroxy-2-(2-methoxymethoxycarbonyl-1-methylethylamino)ethyl]-7-hydroxyindole-2-carboxylic acid methoxymethyl ester.

25. 4-[1-Hydroxy-2-(2-acetoxymethoxycarbonyl-1-methylethylamino)ethyl]-7-hydroxyindole-2-carboxylic acid acetoxymethyl ester.

26. 4-1-Hydroxy-2-[2-(2-dimethylaminoacetoxycarbonyl-1-methylethyl]amino)ethyl-7-hydroxyindole-2-carboxylic acid 2-dimethylaminoethyl ester.

27. 4-1-Hydroxy-2-[2-(2-morpholinoethoxycarbonyl-1-methylethyl]amino)ethyl-7-hydroxyindole-2-carboxylic acid 2-morpholinoethyl ester.

28. 7-Hydroxy-4-(1-hydroxy-2-allylaminoethyl)indole-2-carboxylic acid ethyl ester.

29. 4-1-Hydroxy-2-[2-(4-hydroxyphenyl)-1-methylethylamino]ethyl indole-2-carboxylic acid ethyl ester.

30. 4-[1-Hydroxy-(2-ethoxycarbonyl-1,1-dimethylethylamino)ethyl]-7-hydroxyindole-2-carboxylic acid ethyl ester.

31. N,N'-Hexamethylene-bis-[4-(2-amino-1-hydroxyethyl)-7-hydroxyindole-2-carboxylic acid] ethyl ester.

32. 7-Hydroxy-4-(1-hydroxy-2-tert.-butylaminoethyl)indole-2-carboxylic acid trimethylacetoxymethyl ester.

33. 7-Hydroxy-4-(1-hydroxy-2-tert.-butylaminoethyl)indole-2-carboxylic acid methoxymethyl ester.

34. N-[7-Hydroxy-4-(1-hydroxy-2-tert.-butylaminoethyl)-2-indolecarbonyl] aminoacetic acid methyl ester.

35. [4-(2-tert.-Butylamino-1-hydroxyethyl)-7-hydroxyindole-2-ylmethyl] acetate.

36. 2-(N-tert.-Butylamino)-1-(7-hydroxy-2-hydroxymethylindol-4-yl)ethanol.

37. 2-(N-tert.-Butylamino)-1-(7-hydroxy-2-methylindol-4-yl)ethanol.

38. 4-(1-Hydroxy-2-tert.-butylaminoethyl)-7-hydroxyindole.

39. 7-Hydroxy-4-(1-hydroxy-2-tert.-butylaminoethyl)indole-2-carboxylic acid 2-(morpholino)ethyl ester.

40. Pharmaceutical compositions characterised in that they comprise an indole derivative according to claims 1 to 39.

41. Process for the preparation of indole derivatives of the general formula I, according to claim 1 characterised in that, in known manner,

a) an indole derivative of the general formula II

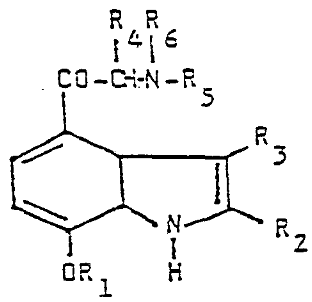

(II)

wherein $R_1$, $R_2$, $R_3$, $R_4$ and $R_5$ have the meanings given in claim 1 and $R_6$ is hydrogen or benzyl, is reduced with optional simultaneous removal of the benzyl group, or

b) in an indole derivative of the general formula I in which $R_1$ is benzyl, the group is removed by hydrogenation, and optionally an ester is saponified or a free acid is esterified and optionally compounds are converted to their salts.

**Revendications**

1. Dérivés de l'indole répondant à la formule générale I :

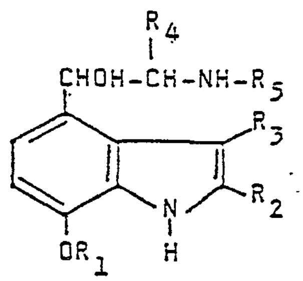

(I)

dans laquelle :

$R_1$ représente un atome d'hydrogène ou un radical méthyle,

$R_2$ représente un atome d'hydrogène, un radical alkyle contenant au plus 4 atomes de carbone, un radical hydroxyméthyle éventuellement estérifié par un alcanoïque renfermant de 1 à 6 atomes de carbone, un radical carboxy, un radical phénoxycarbonyle ou un radical alkylaminocarbonyle, cycloalkylcarbonyle ou alcoxycarbonyle contenant au maximum 8 atomes de carbone et éventuellement interrompu par un atome d'oxygène, un atome d'azote ou un radical carbonyloxy,

$R_3$ représente un atome d'hydrogène ou un radical méthyle,

$R_4$ représente un atome d'hydrogène ou un radical alkyle contenant de 1 à 6 atomes de carbone, et

$R_5$ représente un atome d'hydrogène, un radical phénylalkyle éventuellement interrompu par des radicaux hydroxy, des radicaux alcoxy contenant de 1 à 4 atomes de carbone, des radicaux alkyles contenant de 1 à 4 atomes de carbone ou des atomes d'halogènes, ou un radical alkyle porteur d'un radical alcoxycarbonyle ou cycloalkyl-alcoxycarbonyle éventuellement interrompu par un atome d'oxygène, un atome d'azote ou un radical carbonyloxy, ce radical alkyle $R_5$ contenant en tout au plus 10 atomes de carbone, ou encore $R_5$ représente un radical polyméthylène à au plus 8 atomes de carbone qui unit deux radicaux indoliques répondant à la formule générale Ia :

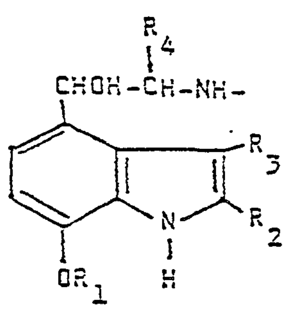

(Ia)

dans laquelle $R_1$, $R_2$, $R_3$ et $R_4$ ont les significations indiquées ci-dessus, ainsi que leurs sels dérivant d'acides inoffensifs du point de vue physiologique et, éventuellement, leurs sels de métaux alcalins et de métaux alcalinoterreux.

2. (Hydroxy-1 amino-2 éthyl)-4 hydroxy-7 indole-carboxylate-2 de méthyle et son chlorhydrate.

3. (Hydroxy-1 méthylamino-2 éthyl)-4 hydroxy-7 indole-carboxylate-2 de méthyle et son chlorhydrate.

4. (Hydroxy-1 isopropylamino-2 éthyl)-4 hydroxy-7 indole-carboxylate-2 de méthyle et son chlorhydrate.

5. Acide d'(hydroxy-1 isopropylamino-2 éthyl)-4 hydroxy-7 indolecarboxylique-2.

6. (Hydroxy-1 tert-butylamino-2 hydroxy-1 éthyl)-4 hydroxy-7 indole-carboxylate-2 de méthyle et son chlorhydrate.

7. Acide (hydroxy-1 tert-butylamino-2 hydroxy-1 éthyl)-4 hydroxy-7 indole-carboxylique-2.

8. (Hydroxy-1 tert-butylamino-2 éthyl)-4 hydroxy-7 indole-carboxylate-2 d'éthyle et son chlorhydrate.

9. (Hydroxy-1 tert-butylamino-2 éthyl)-4 hydroxy-7 indole-carboxylate-2 de n-butyle et son chlorhydrate.

10. (Hydroxy-1 tert-butylamino-2 éthyl)-4 hydroxy-7 indole-carboxylate-2 d'isobutyle et son chlorhydrate.

11. (Hydroxy-1 tert-butylamino-2 éthyl)-4 méthoxy-7 indole-carboxylate-2 de méthyle et son chlorhydrate.

12. [Hydroxy-1 méthyl-1 méthoxycarbonyl-2 éthylamino)-2 éthyl]-4 hydroxy-7 indole-carboxylate-2 d'éthyle et son chlorhydrate.

13. (Hydroxy-1 tert-butylamino-2 propionyl)-4 hydroxy-7 indole-carboxylate-2 d'éthyle et son chlorhydrate.

14. (Hydroxy-1 cyclohexylamino-2 éthyl)-4 hydroxy-7 indole-carboxylate-2 d'éthyle et son chlorhydrate.

15. (Hydroxy-1 cyclopropylamino-2 éthyl)-4 hydroxy-7 indole-carboxylate-2 d'éthyle et son chlorhydrate.

16. Hydroxy-1 [(méthoxy-4 phényl)-2 méthyl-1 éthylamino]-2 éthyl-4 hydroxy-7 indole-carboxylate-2 de méthyle.

17. Hydroxy-1 [(méthoxy-4 phényl)-2 méthyl-1 éthylamino]-2 éthyl-4 hydroxy-7 indole-carboxylique-2.

18. (Hydroxy-1 tert-butylamino-2 éthyl)-4 hydroxy-7 méthyl-3 indole-carboxylate-2 d'éthyle.

19. (Hydroxy-1 tert-butylamino-2 éthyl)-4 hydroxy-7 méthyl-3 indole-carboxylique-2.

20. (Hydroxy-1 tert-butylamino-2 éthyl)-4 hydroxy-7 indole-carboxylate-2 de phényle.

21. (Hydroxy-1 tert-butylamino-2 éthyl)-4 hydroxy-7 indole-carboxylate-2 de diméthylamino-2 éthyle.

22. (Hydroxy-1 tert-butylamino-2 éthyl)-4 hydroxy-7 indole-carboxylate-2 d'acétoxyméthyle.

23. [Hydroxy-1 (méthyl-1 n-butoxycarbonyl-2 éthylamino)-2 éthyl]-4 hydroxy-7 indolecarboxylate-2 de n-butyle.

24. [Hydroxy-1 (méthyl-1 méthoxyméthyloxycarbonyl-2 éthyl-amino)-2 éthyl]-4 hydroxy-7 indole-carboxylate-2 de méthoxyméthyle.

25. [Hydroxy-1 (acétoxyméthyloxycarbonyl-2 éthyl-amino)-2 éthyl]-4 hydroxy-7 indole-carboxylate-2 d'acétoxyméthyle.

26. [Hydroxy-1 méthyl-1-(diméthylamino-2 éthoxycarbonyl)-2 éthylamino]-2 éthyl-4 hydroxy-7 indole-carboxylate-2 de diméthylamino-éthyle.

27. Hydroxy-1 [méthyl-1 (morpholino-2 éthoxycarbonyl)-2 éthyl-amino]-2 éthyl-4 hydroxy-7 indole-carboxylate-2 de morpholino-2 éthyle.

28. Hydroxy-1 allylamino-2 éthyl)-4 hydroxy-7 indole-carboxylate-2 d'éthyle.

29. Hydroxy-1 [méthyl-1 (hydroxy-4 phényl)-2 éthylamino]-2 éthyl-4 indole-carboxylate-2 d'éthyle.

30. [Hydroxy-1 (diméthyl-1,1 éthoxycarbonyl-2 éthylamino)-2 éthyl]-4 hydroxy-7 indole-carboxylate-2 d'éthyle.

31. N,N'-hexaméthylènebis-[(hydroxy-1 amino-2 éthyl)-4 hydroxy-7 indole-carboxylate-2 d'éthyle].

32. (Hydroxy-1 tert-butylamino-2 éthyl)-4 hydroxy-7 indole-carboxylate-2 de triméthylacétoxyméthyle.

33. (Hydroxy-1 tert-butylamino-2 éthyl)-4 hydroxy-7 indole-carboxylate-2 de méthoxyméthyle.

34. N-[(hydroxy-1 tert-butylamino-2 éthyl)-4 hydroxy-7 indolyl-2 carbonyl]-amino-acétate de méthyle.

35. Acétate d'acétoxyméthyl-2 (hydroxy-1 tert-butylamino-2 éthyl)-4 hydroxy-7 indole.

36. (Hydroxyméthyl-2 hydroxy-7 indolyl-4)-1 tert-butylamino-2 éthanol.

37. Acétate de (méthyl-2 hydroxy-7 indolyl-4)-1 tert-butylamino-2 éthanol.

38. (Hydroxy-1 tert-butylamino-2 éthyl)-4 hydroxy-7 indole.

39. (Hydroxy-1 tert-butylamino-2 éthyl)-4 hydroxy-7 indole-carboxylate-2 de morpholino-2 éthyle.

40. Médicaments caractérisés en ce qu'ils contiennent des dérivés de l'indole selon l'une quelconque des revendications 1 à 39.

41. Procédé de préparation de dérivés de l'indole de formule générale I, selon la revendication 1, caractérisé en ce que, en opérant de manière connue :

a) On réduit un dérivé de l'indole répondant à la formule générale II :

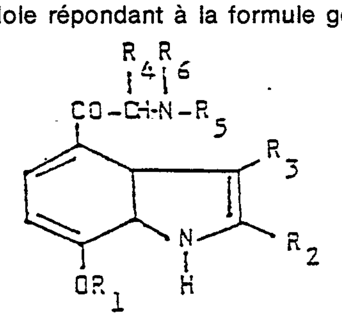

(II)

dans laquelle $R_1$, $R_2$, $R_3$, $R_4$ et $R_5$ ont les significations données à la revendication 1 et $R_6$ représente un atome d'hydrogène ou un radical benzyle, en même temps qu'on élimine éventuellement les radicaux benzyle, ou

b) On dissocie par hydrogénation un dérivé de l'indole de formule générale I dans lequel $R_1$ représente un radical benzyle et, si on le juge bon, on saponifie d'éventuels radicaux d'esters ou on estérifie des radicaux d'acides libres et, si on le désire, on transforme les composés obtenus en leurs sels.